(19) Europäisches Patentamt
European Patent Office
Office européen des brevets

(11) **EP 4 134 976 B1**

(12) **EUROPEAN PATENT SPECIFICATION**

(45) Date of publication and mention
of the grant of the patent:
**05.07.2023 Bulletin 2023/27**

(21) Application number: **21191089.8**

(22) Date of filing: **12.08.2021**

(51) International Patent Classification (IPC):
*G16H 50/20* (2018.01)     *G16H 50/30* (2018.01)
*G16H 50/70* (2018.01)

(52) Cooperative Patent Classification (CPC):
**G16H 50/20; G16H 50/30; G16H 50/70**

(54) **SYSTEM AND METHOD FOR PREDICTING THE RISK OF A PATIENT TO DEVELOP AN ATHEROSCLEROTIC CARDIOVASCULAR DISEASE**

SYSTEM UND VERFAHREN ZUR VORHERSAGE DES RISIKOS EINES PATIENTEN ZUR ENTWICKLUNG EINER ATHEROSKLEROTISCHEN KARDIOVASKULÄREN ERKRANKUNG

SYSTÈME ET PROCÉDÉ POUR PRÉDIRE LE RISQUE D'UN PATIENT DE DÉVELOPPER UNE MALADIE CARDIOVASCULAIRE ATHÉROSCLÉREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.02.2023 Bulletin 2023/07**

(73) Proprietor: **Ada Health GmbH**
**10178 Berlin (DE)**

(72) Inventor: **KESAR, Ajay**
**10435 Berlin (DE)**

(74) Representative: **Bittner, Peter et al**
**Peter Bittner und Partner**
**Herrenwiesenweg 2**
**69207 Sandhausen (DE)**

(56) References cited:
**EP-A1- 2 864 793          EP-B1- 2 864 793**
**CN-B- 106 372 654      RU-C1- 2 573 499**
**RU-C1- 2 654 290**

**Description**

**Technical Field**

The present invention generally relates to electronic data processing, and more particularly, relates to computer-implemented methods, computer program products and systems for predicting the risk of developing an atherosclerotic cardiovascular disease for a patient based on selected risk factors indicating the current medical state of said patient.

**Background**

CVD (cardiovascular disease) is the number one cause of all global death according to the WHO. In 2016, 17.9 million people died of CVD alone, which accounted for 31% of all deaths in that year. CVD is actionable and thus preventable by addressing behavioral risk factors according to a WHO report available at https://www.who.int/en/news-room/fact-sheets/detail/cardiovascular-diseases-(cvds). However, at-risk individuals (patients) require early detection, screening, and health management and there are more than two hundred known potentially relevant risk factors. Behavioral risk factors can be, for example, an unhealthy diet, physical inactivity, smoking, harmful usage of alcohol, and many more lifestyle factors that one is in control of. These behavioral risk factors can lead to intermediate risk factors like primary hypertension or diabetes type 2, that have a direct link to developing CVDs like heart attacks.

In the publication "Cardiovascular disease risk prediction using automated machine learning: A prospective study of 423,604 UK Biobank participants" by Alaa et al. available at https://journals.plos.org/plosone/article?id=10.1371/journal.pone.0213653, an automated machine learning (ML) framework is used to create an ensemble of ML pipelines to retrieve the best performing combination of data preparation and models. The best performing model based on AUROC performance ended up using 473 variables, excluding all patients which had CVD at baseline. This approach is a purely ML-driven approach which focuses only on correlation instead of causation. For example, lab test measurements with no direct link to CVD are taken into account. Additionally, infections and allergies as well as risk factors like fractures, glasses worn, or speed of sound through heel were taken into account although not being relevant to the pathophysiology of CVD. Further, the approach uses a limited CVD definition, neglecting certain entries indicating vascular/heart problems diagnosed by a doctor. It uses an unspecific CVD definition including strokes which are more likely due to a physical trauma (e.g., an accident trauma) instead of atherosclerotic plaque build-up. As a consequence, the risk prediction results provide room for improving the accuracy and reliability of said predictions and do not provide an actionable tool for assessing a current multi-year risk of a patient to develop any atherosclerotic CVD. Further, it is unlikely that the required 473 variables are available for a large number of patients. In the European Patent Application EP 2864793 A1 a method is disclosed for predicting the relative risk of mortality for a patient who already suffers from a cardiovascular disease. However, the relative risk of an already sick patient to die is different from the absolute risk of a healthy patient to develop an atherosclerotic CVD. That is, this method cannot be used for primary prediction and prevention. Russian Patent Application RU 2573499 C1 discloses a method providing a quantitative interpretation (high or low) of the predicted risk of unfavorable outcomes in patients twelve months after unstable angina or acute coronary syndrome in conjunction with type 2 diabetes mellitus. A mathematical model is used to calculate a prediction coefficient of the risk of the unfavorable outcomes using a construction of multi-model prediction based on logistic regression and prediction of differing adverse outcomes after twelve months according to a particular formula which does not take into account human curated risk factors covering multiple biological levels but only blood biomarkers of the patient in the context of developing an atherosclerotic CVD. This method can also not be used for the primary absolute risk prediction of developing an atherosclerotic CVD because the patients already need have had unstable angina or acute coronary syndrome.

**Summary**

It is therefore a problem to provide a system and method for providing accurate and reliable prediction of an individual patient's absolute risk of developing an atherosclerotic cardiovascular disease based on the patient's current medical state, and to provide a basis for identifying potential interventions (proactive treatment proposals) for preventing potential critical CVD clinical outcomes. The current medical state of the patient is reflected by hundreds of known risk factors. In this context it is a technical problem to identify a selection of a minimal viable set with behavioral risk factors for a minimal system with appropriate accuracy in CVD risk prediction for a particular patient. The herein disclosed CVD risk prediction approach focuses on risk prediction with regard to atherosclerotic plaque build-up in arteries. The tendency to build atherosclerotic plaque is determined by multiple factors like genetics, lifestyle and environment. However, the actual risk of developing atherosclerotic plaque might be increased or decreased depending on one's individual lifestyle because this part is actionable and thus preventable through an individual's lifestyle behavior. Thereby it is to be noted that the minimal viable set is a selection from all the latest medically known atherosclerotic CVD risk factors still providing reasonable accuracy while requiring less risk factors. The scope of the invention is thereby defined by the appended claims.

Further, the herein disclosed approach aims at a holistic understanding of a patient's current health status and to quantify said risk of atherosclerotic CVDs by taking into account multiple biological levels (demographics, lifestyle, biomarkers etc.) and human curated knowledge instead of just a single biological level. Multiple biological levels means that a Precision Medicine based approach is applied utilizing multi-omics as well as clinical and lifestyle data to capture all potential interactions or correlations detected between molecules in different biological levels (cf. Collins et al. in "A New Initiative on Precision Medicine", N Engl J Med 2015; 372:793-795, DOI: 10.1056/NEJMp1500523). In the literature biological levels are also referred to as biological layers including but not limited to genomics, epigenomics, transcriptomics, proteomics, and metabolomics. For example, multiple biological levels include, but are not limited to, multi-omics data such as blood samples (e.g., lipidome and proteome), family history (e.g., genome), as well as lifestyle, clinical and environmental data. Most diseases affect complex molecular pathways where different biological layers interact with each other as described by Picar et al. in "Integration strategies of multi-omics data for machine learning analysis", Computational and Structural Biotechnology Journal, Volume 19, 2021, Pages 3735-3746 available at:https://www.sciencedirect.eom/science/article/pii/S2001037021002683#b0025. Multi-omics data broadly cover the data generated from genome, proteome, transcriptome, metabolome, and epigenome. The spectrum of omics can be further extended to other biological data such as lipidome, phosphoproteome, and glycol-proteome. Multi-omics data generated for the same set of samples can provide useful insights into the flow of biological information at multiple levels and thus can help in unraveling the mechanisms underlying the biological condition of interest. Each type of omics data, on its own, typically provides a list of differences associated with the disease. These data can be useful both as markers of the disease process and to give insight as to which biological pathways or processes are different between the disease and control groups. However, analysis of only one data type is limited to correlations, mostly reflecting reactive processes rather than causative ones. Integration of different omics data types is often used to elucidate potential causative changes that lead to disease, or the treatment targets, that can be then tested in further molecular studies (cf. Hasin et al. in "Multi-omics approaches to disease", Genome Biology(Vol. 18, Issue 1), 2017).

When referring to the term "risk prediction" as used hereinafter, the term refers to the absolute risk prediction which is defined as a certain chance of x% for an individual patient to get the disease within a certain time period, given certain findings reflecting the patient's current medical state.

Before going into the details of the solution, it is to be noted that for achieving a high level of accuracy for predicting the risk for a patient to develop a atherosclerotic cardiovascular disease within a predefined time interval, the use of a minimum viable set of human-curated CVD risk factors (mostly behavioral risk factors) covering multiple biological levels of the patient to capture the potential interactions or correlations detected between molecules in different biological levels turned out to be sufficient (cf. AUROC results given below). The minimum viable set includes at least demographic risk factors, biomarker risk factors, comorbidity risk factors, genetic risk factors, and lifestyle risk factors. Said risk factors have been proven as potential causes for developing atherosclerotic plaque build-up. A predictor using the minimal viable set with 25 risk factors as listed in the paragraph after next shows an AUROC of 74,15%. When using a predictor with 203 known risk factors (as listed further down below) only a marginal improvement of the AUROC (75,44%) was achieved.

It was proven that such a holistic view on the behavioral risk factors across all biological levels provides prediction accuracy which is exceeding the prediction accuracy in prior art solutions. For example, the QRISK®3 algorithm (available at https://qrisk.org) calculates a person's risk of developing a heart attack or stroke over the next 10 years and has an AUROC of 72,5%. The approach presented in "The Framingham Heart Study" (by Ralph B. D'AgostinoSr et al, originally published 22 Jan 2008https://doi.org/10.1161/CIRCULATIONAHA.107.699579, Circulation. 2008;117:743-753) has an AUROC of only 68%. The herein disclosed approach takes into account only risk factors relating to the pathophysiology of CVD. Furthermore, in comparison to prior art solutions, the herein disclosed model is interpretable and more compact and thus easier to answer the minimal viable set of 25 questions instead of e.g. 473 questions in a real-world setting. Using this model as part of the herein disclosed system can provide actionable health interventions.

Furthermore, a selection of 25 particular CVD risk factors (out of more than two hundred of known risk factors) already provides a prediction accuracy which is only marginally improved (the AUROC is only improved from 74,15% vs. 75,44%) when taking into account more or all of the remaining known risk factors. The minimal viable set of 25 CVD risk factors includes the following (by risk factor category):

- the demographic risk factors comprising at least the patient's age and gender;

- the biomarker risk factors comprising at least the patient's waist circumference, systolic blood pressure, total Cholesterol, LDL Cholesterol, and total Cholesterol HDL ratio;

- the comorbidity risk factors comprising at least the patient's hypertension, Sleep problems: Not at all, Sleep problems:

Several days, and other Heart Arrhythmias different from Atrial Fibrillation;

- the genetic risk factors comprising at least the patient's familial CVD history; and

- the lifestyle risk factors comprising at least the patient's smoking status, number of cigarettes smoked per day, indicator if alcohol is usually consumed with meals, indicator if alcohol is sometimes consumed with meals, social visits: 2-4 times a week, social visits: about once a week, social visits: about once/month, social visits: almost daily, social visits: once every few months, walking pace: brisk pace, and walking pace: steady average pace, indicator if a self-rated overall health rating is excellent, indicator if a self-rated overall health rating is poor.

Using the minimal viable set instead of all known CVD risk factors has some advantages. Using fewer risk factors simplifies the data collection process to calculate an accurate atherosclerotic CVD risk faster with still reasonable accuracy. Further, data preprocessing and CVD risk calculations require less computing power. When retrieving appropriate interventions, a shorter list with suggested interventions is generated while still ensuring accurate risk predictions. For each CVD risk factor its healthy range is known, and therefore also if its value is elevated or lowered compared to the respective healthy range. Based on this information, the system automatically suggests a meaningful number of personalized interventions to the patient and/or the patient's doctor. The system may even track the patients' progress and continuously refine the suggested interventions based on the patient's current CVD risk and personal preferences and constraints.

In the following, all currently utilized 203 CVD risk factors are listed (separated by ';') without further explanation (including the 25 risk factors of the above minimum viable set). Some risk factors include a general term and a specifying term where the two terms are separated by a ":". A medically trained person will understand the listed CVD risk factors: Hypertension; Age; ldl_cholesterol; High Cholesterol; high_bp; Gender; Other Heart Arrhythmias; total_cholesterol_hdl_ratio; total_cholesterol; Walking pace: Brisk pace; T2D; Sleep problems: Not at all; Weight; Walking pace: Steady average pace; Familial CVD; Smoking; Atrial Fibrillation; Social visits: 2-4 times a week; Alcohol with meals Yes; apolipoprotein b; Diabetes Unspecified; hdl_cholesterol; Social visits: About once a week; Dia bp; No. of cigarettes daily; Obese; CKD; Sys bp; Sleep problems: Several days; Overall health rating: Fair; Alcohol with meals It varies; Overall health rating: Poor; Social visits: Almost daily; Social visits: About once a month; Sleep Apnoea; Alcohol with meals No; BP treatment; Height; T1D; c reactive protein; lipoprotein a; Social visits: Once every few months; red blood cell count; Steroid Medication; Migraines; triglycerides; Ethnicity: Caribbean; Stress; Waist circumference; Commute type: Car/motor vehicle; Current drinker; vitamin d; Sleep problems: Nearly every day; Walk duration in min/day; white blood cell count; Walk duration in min/day smaller 0; Alcohol intake One to three times a month; Sleep problems: More than half the days; High Glucose; Commute type: Public transport; Overall health rating: Good; Employment status: Unable to work because of sickness or disability; Car count: 0.0; Ethnicity: White and Asian; Commute type: Cycle; platelet count; Walking pace: Slow pace; Sleep duration in h/day; Ethnicity: Any other white ; background; Overweight; Accommodation possession: Pay part rent and part mortgage (shared ownership); Accommodation possession: Own with a mortgage; Ethnicity: Indian; Day nap: Sometimes; Ethnicity: Pakistani; Ethnicity: White; Insomnia: Usually; Alcohol intake Never; Rheumatoid Arthritis; Avg income; Overall health rating: Do not know; Poverty; creatinine; Ethnicity: Chinese; Champagne + white wine/week; Social activity: Adult education class; BMI; Ethnicity: White and Black Caribbean; Physical Inactivity; Accommodation type: Prefer not to answer; Ethnicity: Asian or Asian British; Vigorous activity days/week; Social visits: No friends/family outside household; Employment status: Looking after home and/or family; Above 10 min walk days/week; Smoking exposure outside in h/week; Social activity: Pub or social club; glycated haemoglobin; Ethnicity: Irish; Ethnicity: African; Accommodation possession: Do not know; Accommodation type: A house or bungalow; Atypical antipsychotic medication; Employment status: Doing unpaid or voluntary work; Employment status: Unemployed; Social activity: None of the above; Car count: Do not know; Employment status: In paid employment or self-employed; Social visits: Do not know; Bipolar; Accommodation possession: Rent - from local authority - local council - housing association; Employment status: None of the above; Vigorous activity duration in min/day smaller 0; Alcohol intake Once or twice a week; Social visits: Prefer not to answer; Spirits/week; Car count: None; Sleep problems: Prefer not to answer; Ethnicity: Other ethnic group; Arterial stiffness index; Ethnicity: Black or Black British; Red wine/week; Above 10 min walk days/week smaller 0; Social visits: Never or almost never; Social activity: Religious group; Car count: Prefer not to answer; Accommodation possession: Own outright (by you or someone in your household); Inflammation markers; Ethnicity: Do not know; Ethnicity: Prefer not to answer; Social activity: Other group activity; Alcohol with meals Prefer not to answer; Employment status: Retired; Fortified wine/week; Day nap: Prefer not to answer; Car count: 1; Systemic Lupus; Std Sys bp; Psychosis; Accommodation type: Sheltered accommodation; Alcohol with meals Do not know; Ethnicity: Bangladeshi - Caucasian; Social activity: Prefer not to answer; Insomnia: Sometimes; Employment status: Full or part-time student; Accommodation type: A flat - maisonette - apartment; Insomnia: Prefer not to answer; Accommodation possession: Prefer not to answer; Familial diabetes; Ethnicity: Any other Black background; Erectile disfunction diagnosis; Moderate activity duration in min/day smaller 0; Insomnia: Never/rarely; Sleep duration in h/day smaller 0; Vigorous activity days/week smaller 0; Accommodation type: Care home; Overall health rating: Excellent; No. in household; Day nap: Do not know; Unhealthy sleep schedule; Ethnicity: Mixed; Walking pace: None of the above; Familial high bp; Overall health rating: Prefer not to answer; Day nap: Usually; Moderate activity duration in min/day; Accommodation possession: Live in accommodation rent free; Past Smoker; Walking pace: Prefer not to answer; Moderate activity days/week smaller 0; Erectile disfunction treatment; Accommodation possession: None of the above; Beer/week; Townsend; Trouble falling asleep; Accommodation type: None of the above; Schizophrenia; Commute type: None of the above; Alcohol intake Three or four times a week; Car count: 3; Ethnicity: White and Black African; Depression; Smoking exposure home in h/week; Alcohol intake Special occasions only; Accommodation type: Mobile or temporary structure (i.e. caravan); Employment status: Prefer not to answer; Ethnicity: Any other mixed background; Alcohol intake Prefer not to answer; Commute type: Prefer not to answer; Social activity: Sports club or gym; Commute type: Walk; Ethnicity: Any other Asian background; Vigorous activity duration in min/day; Alcohol intake Daily or almost daily; No. of cigarettes daily smaller 0; Ethnicity: British; Accommodation possession: Rent - from private landlord or letting agency; Car count: 2; Moderate activity days/week; Too much sleep; Waking too early; Day nap: Never/rarely.

The above-described technical problem is solved by embodiments of the invention according to the independent claims including a computer system for predicting the absolute risk of a patient to develop an atherosclerotic cardiovascular disease within a predefined time interval, a computer-implemented method which can be executed by said computer system, and a computer program product which, when loaded and executed by the computer system, causes the system to perform said method. Thereby, the predefined time interval specifies a minimum time period which it takes for the formation of said disease. Typically, it takes between 5 and 10 years for an atherosclerotic CVD disease to develop.

In the computer system embodiment, the system is adapted to receive a current set of risk factors associated with the risk of atherosclerotic plaque build-up in the patient's arteries. Thereby, the current set comprises a set of human-curated risk factors covering multiple biological levels including multi-omics data types of the patient to capture the potential interactions or correlations detected between molecules in different layers. It has been found that the human-curated selection of risk factors provides more actionable and interpretable atherosclerotic CVD risk predictions than the pure machine learning based prior art approaches. This is due to the fact that the human-curated set of risk factors takes into account proven causal relationships between the risk factors and the clinical outcomes whereas the pure machine learning based methods learn all kinds of correlations from training data. However, such correlations may be accidental and not related to the respective outcomes or pathophysiology of the diseases. Additionally, in the herein presented application, it was shown that such automatically recognized patterns were not appropriate to provide sufficient actionable and interpretable results with a short set of questions (e.g., questions of the minimal viable set) applicable in daily life to support a patient in suggesting preventive countermeasures to prevent future CVD outcomes. Combining the power of a machine learning predictor with human-curated training data provides better actionability, and reasoning explainability for identifying at-risk patients than pure machine learning based approaches, and is easier to implement in the clinical context.

The human-curated risk factors include at least the 25 risk factors of the minimal viable set in the above-mentioned risk factor categories: demographic risk factors, biomarker risk factors, comorbidity risk factors, genetic risk factors, and lifestyle risk factors to ensure the holistic view on the patient's current medical state by using the current set of risk factors in said categories as test input for a risk prediction predictor when predicting the atherosclerotic CVD risk of a particular patient.

The predictor has been trained with a training data set including a large number of training and testing patient records. Advantageously, the number of training patient records used as training input is orders of magnitude greater than the number of risk factors. The test input data is not contained in the training data sets. However, the structure of the training data (i.e., the risk factors on which the predictor is trained) corresponds to the structure of the test input data (i.e., the risk factors of the current set of said patient). In other words, training data and test input data include the same set of variables. Further, the training data set includes atherosclerotic cardiovascular disease outcomes of the training patients serving as ground truth. Thus, a training data record includes the respective risk factor values of a particular patient at a particular point in time and the information whether this patient has developed any of the atherosclerotic CVD outcome during the predefined training data collection period (the period during which the training data set, i.e., risk factor values and clinical outcomes, was collected). Examples of atherosclerotic cardiovascular disease outcomes are: Coronary/Ischaemic heart disease, Heart attack, Angina, Stroke, Cardiac Arrest, Congestive Heart Failure, Left ventricular failure, Myocardial Infarction, Aortic valve stenosis, Cerebral artery occlusions, Nontraumatic haemorrhages, etc. A more complete list is given in the detailed description.

The training data collection period has at least the length of the predefined time interval for which the predictions are made. It is required that none of the training patients has previously shown an atherosclerotic cardiovascular disease outcome up to the beginning of the training data collection period. In other words, none of the training patients has or had one of the atherosclerotic cardiovascular diseases at the start of the training data collection period. Data records associated with such patients are removed from the training data set before training the predictor. The training process for the predictor is disclosed in great detail in the detailed description. Implementations of the predictor using a Logistic Regression Predictor or a tree-based predictor, have shown the best prediction results. However, the detailed description provides more alternative implementation options for the predictor.

The predictor, after having been trained as described above, then provides in response to the test input (i.e., the current set of risk factor values of the patient), a predicted absolute risk for developing an atherosclerotic cardiovascular disease for said patient within the predefined time interval.

To ensure early identification of at-risk patients with a timely opportunity to apply preventive interactions, the current risk factors of the patient are continuously monitored by receiving updated risk factor values for said patient per predefined monitoring time interval, and then computing an updated risk prediction value using the predictor. A control module of the system ensures that the risk prediction update is triggered in each monitoring time interval.

Depending on the previous risk prediction result (i.e., the result representing the patient's medical state), the monitoring time period may be adjusted. For example, if there are no disease indicators at all for said patient, the monitoring period may be set to three months which may be shortened to for example monthly or even weekly updates in case the system identifies an indication of an increased CVD risk. Such an automatic adjustment of the monitoring time interval can be achieved in an optional system embodiment. In this optional embodiment, the system further includes a mapper module to map the obtained predicted risk value to a corresponding predefined risk level. For example, the mapper may use a look-up table which provides a mapping of certain ranges of the risk factor values in the current set to a respective risk level. Detailed examples are given in the detailed description. In case the corresponding predefined risk level indicates a need for therapeutic intervention, the mapper sends a corresponding notification to the patient, or to a medically trained person taking care of said patient. If such a need for therapeutic intervention is recognized by the system, the monitoring time interval may be shortened. The shortening may depend on the risk level to which the current risk factor values have been mapped.

In a further optional embodiment, in addition to the mapper module, the system had a risk checker module. The risk checker module receives a notification from the mapper in case the corresponding predefined risk level indicates a need for therapeutic intervention. This notification references the risk factor values of the current set of the patient. The risk checker checks for each risk factor of the current set if the respective risk factor exceeds a corresponding maximum value, or falls below a corresponding minimum value (i.e., whether the respective risk factor value is outside a predefined healthy range). If the respective risk factor exceeds the corresponding maximum value, or falls below the corresponding minimum value, the risk checker retrieves one or more suggestions for interventions which, when applied to the patient, are appropriate to reduce the predicted risk for said patient from an intervention database. The intervention database can be an integral component of the computer system or it can be stored at a remote storage location which is accessible by the computer system. The intervention database includes appropriate interventions for particular combinations of risk factor value ranges. Detailed examples are given in the detailed description.

Once the risk checker has retrieved all suggestions for interventions which apply to the patient's current medical state (i.e., the combination of the risk factor values of the current set), the risk checker provides the retrieved one or more suggestions to the patient, and/or to a medically trained person taking care of said patient.

The minimum viable set of risk factors may be extended by further risk factors which leads to slightly improved accuracy values for the CVD risk prediction. For example, the demographic risk factors may further comprise the patient's ethnicity; the biomarkers risk factors may further comprise any of the patient's further weight-related measurements, blood-sample related measurements, heart rate, and glucose; the comorbidity risk factors may further comprise any of the patient's atrial fibrillation, diabetes type 2, diabetes type 1, chronic kidney disease, migraines, rheumatoid arthritis, systemic lupus erythematosus, schizophrenia, bipolar, depression, psychosis, diagnosis or treatment of erectile dysfunction, and atypical antipsychotic medication, inflammation markers, steroids, or medication for any of said comorbidities; the genetic risk factors may further comprise any of the patient's family history of stroke, diabetes, high cholesterol, high blood pressure; and the lifestyle risk factors further comprise any of the patient's stress, overall health rating, physical activity, and sleep status. In addition, environmental risk factors may be used as a further risk category comprising any of the patient's exposure to tobacco smoke, and work, housing, and other socio-economic related factors.

In one embodiment, a computer-implemented method is provided for predicting the absolute risk of a patient to develop an atherosclerotic cardiovascular disease within a predefined time interval. The predefined time interval specifies a minimum time period which it takes for the formation of said disease. The following method steps may be executed by the previously described computer system after a corresponding computer program has been loaded in the memory of the computer system and is executed by at least one processor of the computer system.

The method includes the following steps:

- receiving a current set of risk factors associated with the risk of atherosclerotic plaque build-up in the patient's arteries, wherein the current set comprises a set of human-curated risk factors, with at least demographic risk (210) factors comprising at least the patient's age and gender, biomarker risk factors (220) comprising at least the patient's waist circumference, systolic blood pressure, total Cholesterol, LDL Cholesterol, and total Cholesterol HDL ratio, comorbidity risk factors (230) comprising at least the patient's hypertension, Sleep problems: Not at all, Sleep problems: Several days, and other Heart Arrhythmias different from Atrial Fibrillations, genetic risk factors (240) comprising at least the patient's familial CVD history, and lifestyle risk factors (250) comprising at least the patient's smoking status, number of cigarettes smoked per day, indicator if alcohol is usually consumed with meals, indicator if alcohol is sometimes consumed with meals, social visits: 2-4 times a week, social visits: about once a week, social visits: about once/month, social visits: almost daily, social visits: once every few months, walking pace: brisk pace, and walking pace: steady average pace, indicator if a self-rated overall health rating is excellent, indicator if a self-rated overall health rating is poor;

- providing the current set as test input to a predictor, wherein the predictor has been trained with a training data set including a number of training patient records, the number of training patient records being orders of magnitude greater than the number of risk factors, the training data set including risk factor training variables corresponding to the risk factors of the current set of said patient and serving as training inputs, the training data set further including atherosclerotic cardiovascular disease outcomes of the training patients serving as ground truth, wherein the risk factor training values and the outcomes were obtained over a training data collection period of at least the length of the predefined time interval, and wherein none of the training patients has or had one of the atherosclerotic cardiovascular diseases at the start of the training data collection period; and

- in response to the test input, obtaining from the predictor a predicted risk for developing an atherosclerotic cardiovascular disease for said patient within the predefined time interval.

Optional embodiments of the method may comprise the further steps:

- mapping the obtained predicted risk to a corresponding predefined risk level;

- in case the corresponding predefined risk level indicates a need for therapeutic intervention, notifying the patient and/or a medically trained person accordingly; and/or

- in case the corresponding predefined risk level indicates a need for therapeutic intervention:

    ◦ for each risk factor of the current set:

        ▪ checking if the respective risk factor exceeds a corresponding maximum value, or falls below a corresponding minimum value; and

        ▪ if the respective risk factor exceeds the corresponding maximum value, or falls below the corresponding minimum value, retrieving from an intervention database one or more suggestions for interventions which, when applied to the patient, are appropriate to reduce the predicted risk for said patient;

- providing the retrieved one or more suggestions to the patient and/or to a medically trained person.

- repeating the previous steps once per predefined monitoring time interval for said patient.

Further aspects of the invention will be realized and attained by means of the elements and combinations particularly depicted in the appended claims. It is to be understood that both, the foregoing general description and the following detailed description are exemplary and explanatory only and are not restrictive of the invention as described.

**Short description of the figures**

FIG. 1 illustrates a block diagram of a computer system for predicting the risk of a patient to develop an atherosclerotic cardiovascular disease within a predefined time interval according to an embodiment;
FIG. 2 is a simplified flow chart of a computer-implemented method for predicting the risk of a patient to develop an atherosclerotic cardiovascular disease within a predefined time interval according to an embodiment;
FIG. 3 illustrates various time periods which are relevant in the context of training a predictor and computing CVD risk predictions;
FIG. 4 illustrates a portion of an example questionnaire for collecting individual risk factor values; and

FIG. 5 is a diagram that shows an example of a generic computer device and a generic mobile computer device, which may be used with the techniques described herein.

**Detailed description**

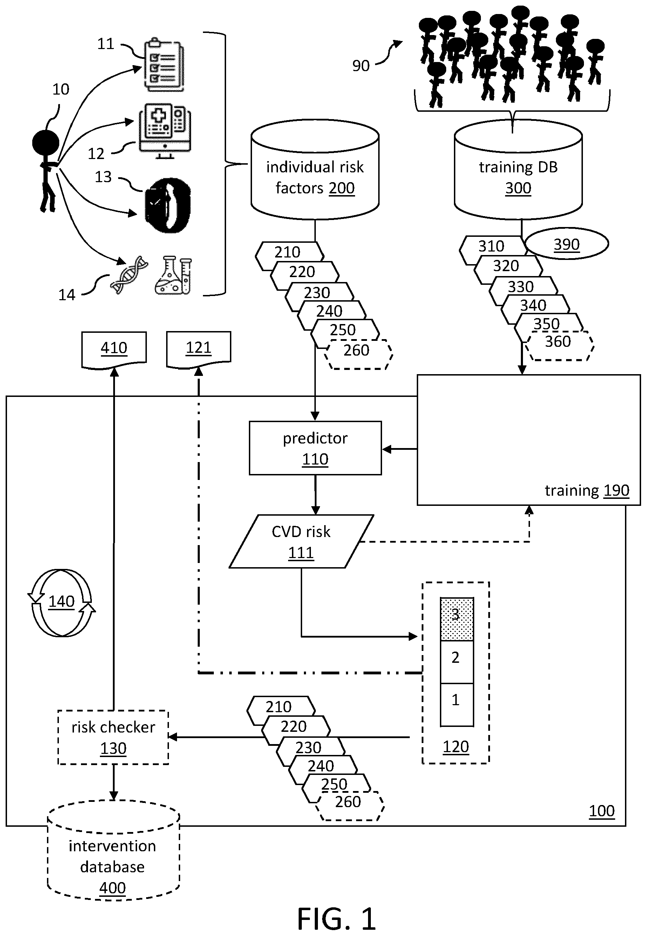

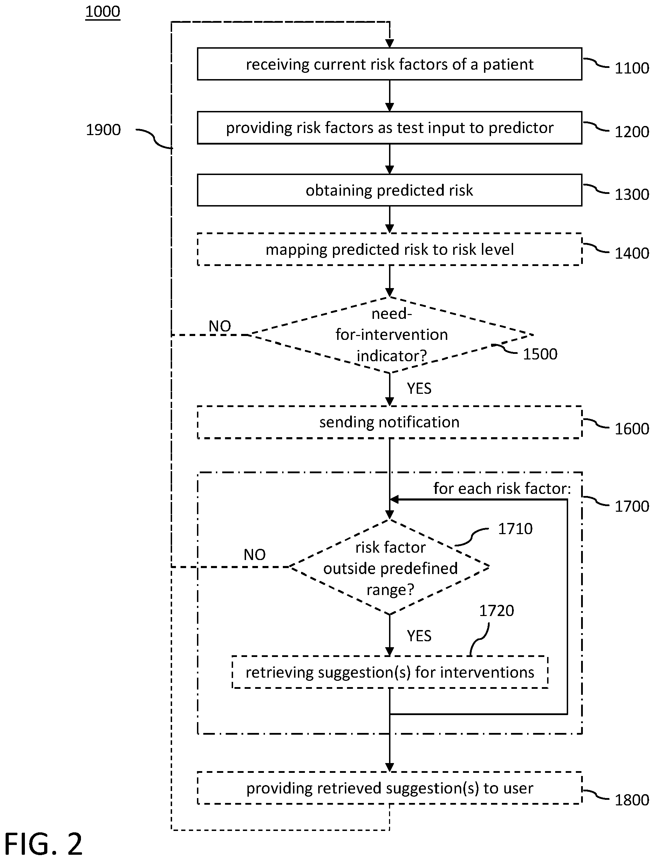

FIG. 1 includes a block diagram of an example embodiment of a computer system 100 for predicting the risk of a patient 10 to develop an atherosclerotic cardiovascular disease within a predefined time interval. The predefined time interval specifies a minimum time period which it takes for the development of said disease. A typical time period for the development of atherosclerotic cardiovascular disease is in the order of 10 years. However, the minimum time period may also be shorter (for example, a 5 years period may still lead to high prediction accuracy). The system 100 of FIG.1 is described in the context of the simplified flow chart of a computer-implemented method 1000 for predicting the risk of a patient 10 to develop an atherosclerotic cardiovascular disease within a predefined time interval as illustrated in FIG. 2. Therefore, the following description of system 100 refers to reference numbers used in FIG. 1 and FIG. 2. The system 100 is thereby configured to execute the method 1000 when loading a respective computer program into a memory of the system and executing said program with processing means of the system.

The system 100 is communicatively coupled via an appropriate interface with a data source 200 providing current individual CVD risk factors of said patient 10 - also referred to as the current set of risk factors. The individual risk factors are associated with the risk of atherosclerotic plaque build-up in the patient's arteries and cover multiple biological levels of the patient to capture the potential interactions or correlations detected between molecules in different biological levels. In other words, the individual risk factors in the current set provide a holistic view on the patient's current medical state with regard to potential future development of CVD. The risk factors of the current set are human-curated in that they have been proven in medical studies as potential causes for developing atherosclerotic plaque build-up. There are hundreds of such risk factors - mostly behavioral risk factors - which can be categorized into the following categories: demographic risk 210 factors, biomarker risk factors 220, comorbidity risk factors 230, family history risk factors 240, and lifestyle risk factors 250. Optionally, the current set may also include environmental risk factors 260.

The current set of risk factors received 1100 by system 100 includes a selection of 25 risk factors (the minimum viable set) out of a plurality of more than 200 potentially relevant risk factors. It is to be noted that the selection of the 25 risk factors is a human curated subset based on the compromise between accurate classifier performance and feasibility. But of course, the values of such risk factors which are used for making CVD risk predictions may be automatically generated by respective sensors or may be automatically retrieved from respective data sources. Thereby, the demographic risk factors 210 comprise at least the patient's age and gender; the biomarker risk factors 220 comprise at least the patient's waist circumference, systolic blood pressure, total Cholesterol, LDL Cholesterol, and total Cholesterol HDL ratio; the comorbidity risk factors 230 comprise at least the patient's hypertension, Sleep problems: Not at all, Sleep problems: Several days, and other Heart Arrhythmias different from Atrial Fibrillation; the genetic risk factors 240 comprise at least the patient's familial CVD history; and the lifestyle risk factors 250 comprise at least the patient's smoking status, number of cigarettes smoked per day, indicator if alcohol is usually consumed with meals, indicator if alcohol is sometimes consumed with meals, social visits: 2-4 times a week, social visits: about once a week, social visits: about once/month, social visits: almost daily, social visits: once every few months, walking pace: brisk pace, and walking pace: steady average pace, indicator if a self-rated overall health rating is excellent, indicator if a self-rated overall health rating is poor.

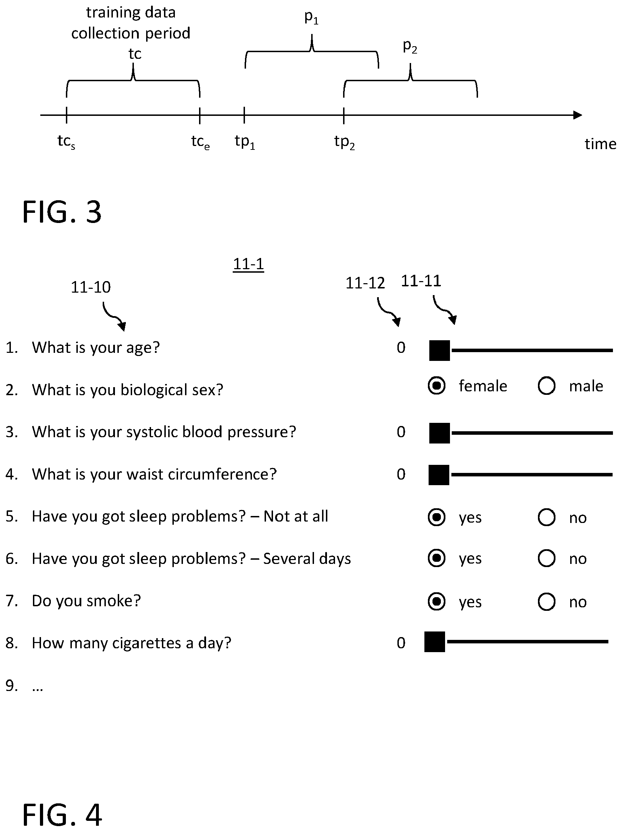

The data source 200 can receive the risk factors of the patient's current set from different sources. For example, risk factors such as the patient's smoking or sleeping behavior may be collected via a questionnaire 11 provided to the patient (e.g., via a respective app on a computing device). An extract of an example questionnaire 11-1 is shown in FIG. 4. The questionnaire 11-1 may be presented as an online questionnaire where the patient can answer questions 11-10 of the minimal viable set using interaction objects 11-11. In the example, the interaction objects for inputting number values 11-12 (questions 1, 3, 4, 8) may be implemented as slider bars. The interaction objects for inputting Boolean values (questions 2, 5, 6, 7) may be implemented as radio buttons.

Some risk factors may be continuously monitored by the patient by wearing a corresponding wearable device 13 with appropriate sensors. For example, wearables and smart devices at home with sensors, such as smartwatch, smartphone, smart scale, blood pressure cuff, all-in-one home testing devices, user-facing radar sensor on bedside device or wearable fitness trackers can provide certain CVD risk factors of the patient in real-time (e.g., walking pace, sleep problems, atrial fibrillation or other heart arrhythmias, etc.).

Intelligent algorithms, for example in a companion app, can derive risk factor values from stored data, smartphone cameras, available sensors in smartphone and wearables, calendar, intelligent computer vision algorithm deducing blood pressure values from smartphone camera, smart smoking detection algorithm, social visits detection by evaluating calendar, sleep tracking algorithm, heart arrhythmia classifier algorithm, diabetes classifier algorithm.

Other CVD risk factors (e.g., Total cholesterol, Hdl cholesterol, Ldl cholesterol, Apolipoprotein b, Total cholesterol Hdl ratio, High Cholesterol, Type 2 Diabetes) may be provided to the individual risk factors data source 200 via lab tests 14 from laboratories for blood samples or using appropriate home test kits.

Another source of individual risk factors of the patient 10 can be electronic health records (EHR) of said patient and family members (e.g., ECG, blood pressure or other vital data, comorbidity data) from hospitals, GPs, cardiologists, psychologists, or lifestyle physicians.

The following list shows an example of how to provide details about the data types of the risk factors of the minimum viable set, and about their meaning, their sources and harmonization steps (a skilled person may choose different representations, formats or data sources):

1. Age: chronological age in years as float, from EHR from hospital or GP, or fallback questionnaire in companion app

2. Gender: biological sex, female = 0.0, male = 1.0, from EHR from hospital or GP, or fallback questionnaire in companion app

3. Social visits: About once a week: boolean value encoded as integer, yes = 1, no = 0, visiting friends or family or have them visit about 1 time a week on average, including meeting with friends or family in environments outside of the home such as in the park, at a sports field, at a restaurant or pub, from EHR from hospital, GP, cardiologist or psychologist or from questionnaire in companion app, calendar

4. Social visits: 2-4 times a week: boolean value encoded as integer, yes = 1, no = 0, visiting friends or family or have them visit 2-4 times a week on average, including meeting with friends or family in environments outside of the home such as in the park, at a sports field, at a restaurant or pub, from EHR from hospital, GP, cardiologist or psychologist or from questionnaire in companion app, calendar

5. Social visits: About once a month: boolean value encoded as integer, yes = 1, no = 0, visiting friends or family or have them visit about 1 time a month on average, including meeting with friends or family in environments outside of the home such as in the park, at a sports field, at a restaurant or pub, from EHR from hospital, GP, cardiologist or psychologist or from questionnaire in companion app, calendar

6. Social visits: Almost daily: boolean value encoded as integer, yes = 1, no = 0, visiting friends or family or have them visit nearly every day on average, including meeting with friends or family in environments outside of the home such as in the park, at a sports field, at a restaurant or pub, from EHR from hospital, GP, cardiologist or psychologist or from questionnaire in companion app, calendar

7. Social visits: Once every few months: boolean value encoded as integer, yes = 1, no = 0, visiting friends or family or have them visit once every few months on average, including meeting with friends or family in environments outside of the home such as in the park, at a sports field, at a restaurant or pub, from EHR from hospital, GP, cardiologist or psychologist or from questionnaire in companion app, calendar

8. Familial CVD: Family history of relevant CVDs diagnosed in father, mother or siblings like ischaemic heart disease, heart disease or other CVDs, boolean value encoded as integer, yes = 1, no = 0, retrieved from EHR from patient and family from hospital, GP or cardiologist or from questionnaire in companion app

9. Smoking: boolean value encoded as integer, yes = 1, no = 0, current smoker on most or all days or occasionally, retrieved from EHR from hospital, GP, cardiologist or lifestyle physician or from questionnaire in companion app or from air sensor in smartwatch and smart smoking detection algorithm in companion app

10. No. of cigarettes daily: Integer value >= 0, number of daily smoked tobacco cigarettes on average, from questionnaire in companion app or from air sensor in smartwatch and smart smoking detection algorithm in companion app, fix outliers by setting all values above 60 to 60

11. Alcohol with meals: Yes: boolean value encoded as integer, alcohol usually consumed with meals = 1, alcohol not usually consumed with meals = 0, retrieved from EHR from hospital, GP, cardiologist or lifestyle physician or from questionnaire in companion app

12. Alcohol with meal: It varies: boolean value encoded as integer, alcohol sometimes consumed with meals = 1, alcohol not sometimes consumed with meals = 0, retrieved from EHR from hospital, GP, cardiologist or lifestyle physician or from questionnaire in companion app

13. Walking pace: Steady average pace: boolean value encoded as integer, yes = 1, no = 0, defined as between 3-4 miles per hour, from EHR from psychologist, from questionnaire in companion app or measured through accelerometer & gyroscope sensor and enhanced with altimeter and GPS sensors in smartphone, measures through accelerometer & gyroscope sensor in smartwatch

14. Walking pace: Brisk pace: boolean value encoded as integer, yes = 1, no = 0, defined as more than 4 miles per hour, from questionnaire in companion app or measured through accelerometer & gyroscope sensor and enhanced with altimeter and GPS sensors in smartphone, measures through accelerometer & gyroscope sensor in smartwatch

15. Systolic blood pressure: in mmHG as float, from EHR from hospital, GP or cardiologist, blood pressure cuff at home or smartwatch connected to companion app or fallback questionnaire in companion app or intelligent computer vision algorithm deducing blood pressure values from smartphone camera

16. Hypertension: boolean value encoded as integer, yes = 1, no = 0, diagnosis retrieved from EHR from hospital, GP or cardiologist, blood pressure cuff at home or smartwatch connected to companion app or fallback questionnaire in companion app or intelligent computer vision algorithm deducing blood pressure values from smartphone camera

17. Other Heart Arrhythmias: boolean value encoded as integer, yes = 1, no = 0, a detected heart arrhythmia other than atrial fibrillation, from EHR from hospital, GP, cardiologist or lifestyle physician or from questionnaire in companion app or collected from smartphone via connected smartwatch with ECG or PPG sensor and classified by smartphone operating system or classified using own heart arrhythmia classifier algorithm in companion app connected to smartwatch with ECG or PPG sensor or from smartphone camera

18. Sleep problems: Not at all: boolean value encoded as integer, yes = 1, no = 0, no trouble falling or staying asleep or sleeping too much, from EHR from GP or lifestyle physician or from questionnaire in companion app or measured through accelerometer & gyroscope sensor in smartphone or from smartwatch or user-facing radar sensor on bedside device combined with sleep tracking algorithm

19. Sleep problems: Several days: boolean value encoded as integer, yes = 1, no = 0, Trouble falling or staying asleep or sleeping too much several days per week, from EHR from GP or lifestyle physician or from questionnaire in companion app or measured through accelerometer & gyroscope sensor in smartphone or from smartwatch or user-facing radar sensor on bedside device combined with sleep tracking algorithm

20. Waist circumference: in cm as float, from EHR from hospital, GP, cardiologist or lifestyle physician, smart scale at home connected to companion app or fallback questionnaire in companion app

21. Overall health rating: Excellent: boolean value encoded as integer, yes = 1, no = 0, excellent self-rated general overall health rating, from EHR from hospital, GP, cardiologist or lifestyle physician, or fallback questionnaire in companion app

22. Overall health rating: Poor: boolean value encoded as integer, yes = 1, no = 0, poor self-rated general overall health rating, from EHR from hospital, GP, cardiologist or lifestyle physician, or fallback questionnaire in companion app

23. Total cholesterol: in mmol/L as float, from blood sample, from EHR from hospital, GP, cardiologist, lab, biomarker test kit provider or from a cholesterol sensor connected to the companion app, or from questionnaire in companion app

24. Ldl cholesterol: in mmol/L as float, from blood sample, from EHR from hospital, GP, cardiologist, lab, biomarker test kit provider or from a cholesterol sensor connected to the companion app, or from questionnaire in companion app

25. Total cholesterol Hdl ratio: float, total cholesterol divided by hdl cholesterol, from already existing values or from blood sample, from EHR from hospital, GP, cardiologist, lab, biomarker test kit provider or from a cholesterol sensor connected to the companion app, or from questionnaire in companion app

Once the system 100 has received the current set of the patient's individual risk factors, the current set is provided 1200 to a predictor 110 of the system as a test input. The predictor 110 has been trained by using a training module 190 to provide, in response to the test input, a predicted risk value 111 for developing an atherosclerotic cardiovascular disease for said patient 10 within the predefined time interval. Thereby, while the predictor is learning, it may recognize patterns which are distributed over multiple biological levels. Instead of only looking at a single level (e.g., family history/genomics), a holistic view of the disease is provided. The training module 190 can be an integral part of the system 100, or it may be provided by another computer system which is communicatively coupled with system 100.

The predictor 110 has been trained with a training data set including a large number of training patient records from training patients 90. Typically, the training data set is provided by a training database 300 which is accessible by the training module 190. An example of such a training database is the UK BioBank (accessible via https://www.ukbiobank.ac.uk/enable-your-research) which provides a training data set with more than 500.000 patient data records. In one embodiment, the following instances of the UK BioBank http://biobank.ndph.ox.ac.uk/showcase/instance.cgi?id=2 was used for the training of the predictor. Other training data sets may be used if they include a number of training patient records which is orders of magnitude greater than the number of risk factors. The training data set includes risk factor training values 310 to 350 in the same risk factor categories as the test input. In other words, training data and the current set of the patient have the same set of variables (i.e., the minimal viable set of risk factors). In FIG. 1, the hexagons 310 to 360 and 210 to 260 represent the risk factor values in the respective categories for the training input data and the test input, respectively. The training data set 300 further includes atherosclerotic cardiovascular disease outcomes 390 of the training patients 90. These outcomes serve as the ground truth when training the predictor 110. All ICD-10 and ICD-9 atherosclerotic CVDs are taken into account, and not just a subset. Further, CVDs which cannot be influenced by the patient's lifestyle habits were not taken into account (e.g., pregnancy, congenital and accident-related CVDs were excluded). In other words, the outcomes serving as ground truth are also human curated. All atherosclerotic CVDs are summarized into one category distinguishing whether a training patient did or did not develop a atherosclerotic CVD within the predefined time period. The training data is entirely based on objective data relying on real medical and GP records with medical-grade.

Turning briefly to FIG. 3, the risk factor training values and the outcomes were obtained over a training data collection period tc of at least the length of the predefined time interval. In FIG. 3, after the end $tc_e$ of the training data collection period tc, and after the predictor has been trained with the collected training data, the predictor can make risk predictions for individual patients at any point in time based on the respective current test inputs. In the example of FIG.

3, predictions are made at time points $tp_1$ and $tp_2$ for time periods $p_1$ and $p_2$ which have the length of the predefined time interval and must not be longer than the training data collection period tc. It is important that none of the training patients 90 has shown an atherosclerotic cardiovascular disease outcome up to the beginning $tc_s$ of the training time data collection period tc.

In general, the number of training patient records with positive outcomes (in an example implementation over 28.000 records) should be much larger than the unique number of outcomes (in the example implementation 193 total or 125 unique CVD outcomes) to provide a lot of training data records with positive and negative outcomes based on different risk factor constellations. In the example implementation the following absolute and relative number of entries with a positive and negative CVD occurrence in the 10 year assessment period were observed: 28,561 people did develop an atherosclerotic CVD (6.1%) and 435,986 people did not develop an atherosclerotic CVD (93,9%) in those 10 years with using all 203 risk factors.

If there is sufficient continuous data of a lot of training patients available, shorter prediction time periods might be possible, but these datasets are virtually non-existent. On the other hand, from a medical point of view, cardiovascular diseases usually develop over a longer period of time (decades) as it takes a while for arteries to block up with plaque. For this reason, they are more common for people aged 40 and up than for younger people. Generally, 10 years are preferable and currently, the most used prediction timeframe for CVD risk prediction.

The following list of CVD outcomes (separated by ';') were taken into account with the training dataset of the UK BioBank using ICD-10 or ICD-9 codes or other data encodings (known by the skilled person) for the respective diseases:

Data Coding 19 Codes: ICD-10 Codes; F01.9 Vascular dementia, unspecified; F01.8 Other vascular dementia; F01.3 Mixed cortical and subcortical vascular dementia; F01.2 Subcortical vascular dementia; F01.1 Multi-infarct dementia; F01.0 Vascular dementia of acute onset; G45.0 Vertebro-basilar artery syndrome; G45.1 Carotid artery syndrome (hemispheric); G45.3 Amaurosis fugax; G45.4 Transient global amnesia; G45.8 Other transient cerebral ischaemic attacks and related syndromes; G45.9 Transient cerebral ischaemic attack, unspecified; 111.0 Hypertensive heart disease with (congestive) heart failure; 111.9 Hypertensive heart disease without (congestive) heart failure; 113.0 Hypertensive heart and renal disease with (congestive) heart failure; I13.1 Hypertensive heart and renal disease with renal failure; 113.2 Hypertensive heart and renal disease with both (congestive) heart failure and renal failure; I13.9 Hypertensive heart and renal disease, unspecified; I20.0 Unstable angina; I20.8 Other forms of angina pectoris; I20.9 Angina pectoris, unspecified; I21.0 Acute transmural myocardial infarction of anterior wall; I21.1 Acute transmural myocardial infarction of inferior wall; I21.2 Acute transmural myocardial infarction of other sites; I21.3 Acute transmural myocardial infarction of unspecified site; I21.4 Acute subendocardial myocardial infarction; I21.9 Acute myocardial infarction, unspecified; I22.0 Subsequent myocardial infarction of anterior wall; I22.1 Subsequent myocardial infarction of inferior wall; Subsequent non-ST elevation (NSTEMI) myocardial infarction; I22.8 Subsequent myocardial infarction of other sites; I22.9 Subsequent myocardial infarction of unspecified site; I23.0 Haemopericardium as current complication following acute myocardial infarction; I23.1 Atrial septal defect as current complication following acute myocardial infarction; I23.2 Ventricular septal defect as current complication following acute myocardial infarction; I23.3 Rupture of cardiac wall without haemopericardium as current complication following acute myocardial infarction; I23.4 Rupture of chordae tendineae as current complication following acute myocardial infarction; I23.5 Rupture of papillary muscle as current complication following acute myocardial infarction; I23.6 Thrombosis of atrium, auricular appendage and ventricle as current complications following acute myocardial infarction; I23.8 Other current complications following acute myocardial infarction; I24.0 Coronary thrombosis not resulting in myocardial infarction; I24.8 Other forms of acute ischaemic heart disease; I24.9 Acute ischaemic heart disease, unspecified; I25.0 Atherosclerotic cardiovascular disease, so described; I25.1 Atherosclerotic heart disease; I25.2 Old myocardial infarction; I25.3 Aneurysm of heart; I25.4 Coronary artery aneurysm; I25.5 Ischaemic cardiomyopathy; I25.6 Silent myocardial ischaemia; I25.8 Other forms of chronic ischaemic heart disease; I25.9 Chronic ischaemic heart disease, unspecified; I34.0 Mitral (valve) insufficiency; I35.0 Aortic (valve) stenosis; I35.1 Aortic (valve) insufficiency; I35.2 Aortic (valve) stenosis with insufficiency; I37.1 Pulmonary valve insufficiency; I42.1 Obstructive hypertrophic cardiomyopathy; I42.6 Alcoholic cardiomyopathy; I44.0 Atrioventricular block, first degree; I44.1 Atrioventricular block, second degree; I44.2 Atrioventricular block, complete; I46.0 Cardiac arrest with successful resuscitation; I46.1 Sudden cardiac death, so described; I46.9 Cardiac arrest, unspecified; I50.0 Congestive heart failure; I50.1 Left ventricular failure; I50.2 Systolic (congestive) heart failure; I50.3 Diastolic (congestive) heart failure; I50.4 Combined systolic (congestive) and diastolic (congestive) heart failure; I50.8 Other heart failure; I50.9 Heart failure, unspecified; I51.3 Intracardiac thrombosis, not elsewhere classified; I51.6 Cardiovascular disease, unspecified; I51.7 Cardiomegaly; I62.1 Nontraumatic extradural haemorrhage; I62.9 Intracranial haemorrhage (nontraumatic), unspecified; I63.0 Cerebral infarction due to thrombosis of precerebral arteries; I63.1 Cerebral infarction due to embolism of precerebral arteries; I63.2 Cerebral infarction due to unspecified occlusion or stenosis of precerebral arteries; I63.3 Cerebral infarction due to thrombosis of cerebral arteries; I63.4 Cerebral infarction due to embolism of cerebral arteries; I63.5 Cerebral infarction due to unspecified occlusion or stenosis of cerebral arteries; I63.6 Cerebral infarction due to cerebral venous thrombosis, nonpyogenic; I65.0 Occlusion and stenosis of vertebral artery; I65.1 Occlusion and stenosis of basilar artery;

I65.2 Occlusion and stenosis of carotid artery; I65.3 Occlusion and stenosis of multiple and bilateral precerebral arteries; I65.8 Occlusion and stenosis of other precerebral artery; I65.9 Occlusion and stenosis of unspecified precerebral artery; I66.0 Occlusion and stenosis of middle cerebral artery; I66.1 Occlusion and stenosis of anterior cerebral artery; I66.2 Occlusion and stenosis of posterior cerebral artery; I66.3 Occlusion and stenosis of cerebellar arteries; I66.4 Occlusion and stenosis of multiple and bilateral cerebral arteries; I66.8 Occlusion and stenosis of other cerebral artery; I66.9 Occlusion and stenosis of unspecified cerebral artery; I67.0 Dissection of cerebral arteries, nonruptured; I67.1 Cerebral aneurysm, nonruptured; I67.2 Cerebral atherosclerosis; I67.3 Progressive vascular leukoencephalopathy; I67.4 Hypertensive encephalopathy; I67.5 Moyamoya disease; I67.6 Nonpyogenic thrombosis of intracranial venous system; I67.7 Cerebral arteritis, not elsewhere classified; I67.8 Other specified cerebrovascular diseases; I67.9 Cerebrovascular disease, unspecified; I68.0 Cerebral amyloid angiopathy; I68.8 Other cerebrovascular disorders in diseases classified elsewhere; I69.8 Sequelae of other and unspecified cerebrovascular diseases; Data Coding 87 Codes: ICD-9 Codes; Diseases of mitral and aortic valves; Acute myocardial infarction; Postmyocardial infarction syndrome; Other acute and subacute forms of ischaemic heart disease; Old myocardial infarction; Angina pectoris; Coronary atherosclerosis; Aneurysm of heart; Other specified forms of chronic ischaemic heart disease; Chronic ischaemic heart disease, unspecified; Aortic valve disorders; Atrioventricular block, complete; Cardiac arrest; Congestive heart failure; Left heart failure; Cardiovascular disease, unspecified; 4320 Nontraumatic extradural haemorrhage; 4331 Occlusion and stenosis of carotid artery; 4339 Occlusion and stenosis of precerebral arteries, unspecified; 4349 Occlusion of cerebral arteries, unspecified; 4359 Transient cerebral ischaemia; 4370 Cerebral atherosclerosis; 4371 Other generalized ischaemic cerebrovascular disease; 4372 Hypertensive encephalopathy; 4373 Cerebral aneurysm, nonruptured; Data Coding 100605 Codes; Heart Attack; Angina; Stroke; Data Coding 6 Codes: self reported diseases; aortic stenosis; aortic aneurysm; aortic dissection; ischaemic stroke; aortic aneurysm rupture; aortic valve disease; heart failure/pulmonary odema; mitral regurgitation / incompetence; mitral valve disease; aortic regurgitation / incompetence; heart attack/myocardial infarction; stroke; arterial embolism; transient ischaemic attack (tia)

For this reason, in the implementation of the training module 190 using the UK BioBank, initially all medical entries of patients which already had or have one of the atherosclerotic CVDs at the start of the (10 years) training data collection period were erased from the overall dataset of the UK BioBank. In other words, the patient records in the UK BioBank related to patients who showed an atherosclerotic cardiovascular disease outcome up to the beginning $tc_s$ of the training time data collection period tc were filtered out and did not become part of the training DB 300. By removing patients with a prior CVD at the beginning training data collection period a clean baseline is created.

Further, for the filtered training DB 300, the remaining data were split into a training and testing set. In other words, the remaining data was split into a 70% training subset consisting of the binary tuple (X_train, y_train) and a 30% test subset with the binary tuple (X_test, y_test), where X denotes the risk factor input values and y the CVD outcomes, y, the CVD outcomes, is a singular column consisting of zeros '0' and ones '1' only. A '1' indicates that at any time during the data collection period (e.g., during a 10 year assessment period), any one or more of the atherosclerotic CVDs occurred.

In a normalization step, mean normalization was performed, where the predictor learns the parameters on X_train and applies them to X_train and X_test. This step reduces the range of feature values and allows for better results. Features are standardized by removing the mean and scaling to unit variance. The normalized value of a sample x is calculated as: $z = (x - u) / s$ where u is the mean of the training samples and s is the standard deviation of the training samples. Centering and scaling happen independently on each feature by computing the relevant statistics on the samples in the training set X_train. Mean and standard deviation are then stored to be used on later data.

In an imputation step, missing values were completed by using a mean value imputation strategy. The learned parameters were applied on X_train and X_test. Missing values were replaced using the mean along each feature column.

When training the predictor 110, a Logistic Regression Classifier was found as a preferred embodiment. However, the skilled person may use other predictor types instead. The logistic regression classifier was trained on the training data by providing it the transformed X_train and y_train datasets and using an implementation of sklearn available at: https://scikit-learn.org/stable/modules/generated/sklearn.linear_model.LogisticRegression.html

The training parameters of Logistic Regression were set to the following:

1. 'C': 1.0, the Inverse of regularization strength was set to 1.0

2. 'class_weight': None, all classes are supposed to have weight one.

3. 'dual': False, we use the primal formulation

4. 'fit_intercept': True, specifies if a constant (bias or intercept) should be added to the decision function

5. 'intercept_scaling': 1, x becomes [x, self.intercept_scaling], i.e. a "synthetic" feature with constant value equal to intercept_scaling is appended to the instance vector. The intercept becomes intercept_scaling * synthetic_feature_weight.

6. 'l1_ratio': None, do not use elastic net penalty

7. 'max_iter': 1000, use a maximum number of 1000 iterations taken for the solver to converge

8. 'multi_class': 'auto',

9. 'n_jobs': None, ignored number of CPU cores to use

10. 'penalty': 'l1', l1 regularization as norm for the penalization

11. 'random_state': 42, to shuffle the data

12. 'solver': saga, solver algorithm for the optimization problem

13. 'tol': 0.0001, our tolerance for the stopping criteria of the training is set to 1e-4

14. 'verbose': 0, set verbose to any positive number for verbosity

15. 'warm_start': False, do not reuse the solution of the previous call to fit as initialization and just erase the previous solution.

The predictor was trained on only the 70% training data set, which consists of risk factor inputs (X_train) and CVD outcomes outputs (y_train). The input to the predictor includes two items:

1st Item 1 X_train: the risk factor values at the start of the data collection period tc when the values where first recorded (for example, a date between 2006-2010 in the used training dataset per patient)

2nd Item 2 y_train: the atherosclerotic CVD outcomes, specifying if any atherosclerotic CVD occurred during the data collection period (e.g., during a 10 year assessment period).

The training data includes at least the CVD risk factor values of the minimum viable set and the CVD outcomes from the whole 10-year follow-up period.

The test data, which consists of 30% of all patient data. For testing, the class labels y_test were predicted for all test data samples X_test from the test data set.

For performance assessment, numerous performance metrics were calculated and stored, including: accuracy, probability predictions, false positive rate, true positive rate, Area Under the Receiver Operating Characteristic Curve (AUROC) from prediction scores, precision, recall, f1 score, support, mispredictions. They help to evaluate the safeness, usability and improvement of the current classifier training on the latest available data of all patients to predict their 10-year CVD risk.

The mathematical formulas in Box F1 are calculated by the predictor for every patient to predict the risk of someone developing a behavioral risk within the predefined timer interval (in the example implementation: within the next 10-years):

- **The logit** $f(x_1, x_2) = b_0 + b_1 x_1 + b_2 x_2$

- **The probabilities** $p(x_1, x_2) = 1 / (1 + \exp(-f(x_1, x_2)))$

$$(F1)$$

Each input risk factor value is normalized with the same parameters as learned per each risk factor during training of the classifier (predictor) model. The resulting probability p is the CVD risk, $x_i$ are the risk factor values and $b_i$ the respective coefficients of the features learned from training. Each input risk factor value is converted to a numerical value, then the learned mean value u is subtracted and then the value is divided by the learned standard deviation s and then multiplied by its coefficient value b. b0 is the learned bias or intercept value.

In alternative implementations, XG Boost (which is a collection of decision trees) was used. Decision trees also explain their reasoning but can get quite complex and not humanly interpretable anymore. For this reason, the Logistic Regression classifier can be advantageous. Neural networks are generally considered "black-boxes" as they don't provide reasoning as to how they come to conclusions. For this reason, they are less advantageous to be used as a predictor in the herein disclosed approach.

Table 1 shows a comparison of the AUROC performance of different algorithms which were used for the implementation of the predictor.

*Table 1 - algorithm performance*

| Algorithm Name & Best Parameter Settings | Auroc |
| --- | --- |
| Extreme Gradient Boosting: subsample: 0.8, n_estimators: 1500, max_depth: 5, learning_rate: 0.01, gamma: 1, colsample_bytree: 0.6 | 0.7572576968 |
| Voting Classifier 1: LogRegL1 + Random Forest 1000 | 0.7559084444 |
| Logistic Regression, L1 regularization Saga | 0.7544434059 |
| Logistic Regression Elastic Net: L1 Ratio 0.05 | 0.754439278 |
| Logistic Regression, no regularization | 0.7544384773 |
| Multi-layer Perceptron SGD: hidden_layer_sizes: (200, 150), learning_rate_init: 0.1 | 0.7477926157 |
| Voting Classifier 2: LogRegL1 + Random Forest 20 | 0.7313623472 |
| Stochastic Gradient Descent Classifier | 0.6909589378 |
| Random Forest Classifier: n_estimators=20 | 0.6690304885 |
| Decision Tree Classifier | 0.5295528575 |

For assessing the algorithm performance multiple metrics were used and compared with regard to how already available and validated CVD risk prediction models performed on the same dataset. With regard to the performances of already available methods it should be taken into account that they have been trained, tested and tuned on different datasets with different objectives. Performance metrics for picking an algorithm comprise: AUROC, FPR, TPR, FNR, TNR, absolute number of wrong & correct predictions, accuracy, f1 score, precision, recall, training time, how generalizable does it perform when trained purely on Caucasians and tested on only non-Caucasians. Implementations with an AUROC above 70% are preferable to prior art solutions.

In an optional embodiment, the system 100 further has a control module 140 to trigger the risk prediction by the predictor once per predefined monitoring time interval for a patient who is to be continuously monitored. The length of the monitoring time interval may be adjusted depending on the clinical state of said patient. For example, when the patient has a low CVD risk, the monitoring may be performed only once a year. In cases with high CVD risk values, the risk prediction may be repeated 1900 monthly or even weekly.

In one embodiment, the system 100 further has a mapper module 120 to map 1400 the predicted CVD risk value 111 to a corresponding predefined risk level. In the example of FIG. 1, the mapper 120 supports three risk levels 1, 2 and 3. Risk level 1 may stand for low CVD risk, level 2 for medium CVD risk and level 3 for high CVD risk. In the example, the risk value 111 is mapped to risk level 3 (dotted background). The mapper 120 checks 1500 if the predefined risk level 3 indicates a need for therapeutic intervention (as it would be the case for a high-risk level). In such a case, the system sends 1600 a corresponding notification 121 to the patient 10 and/or a medically trained person taking care of said patient. Such notification triggers the patient and/or the doctor to look into potential preventive actions which are appropriate to reduce the CVD risk for said patient in the long run. Corresponding therapeutic decisions are usually made by the medically trained person based on his/her personal experience.

In one embodiment, the system 100 supports the decision for appropriate interventions. In this embodiment, again the mapper module 120 configured maps the obtained predicted risk value 111 to a corresponding predefined risk level 1, 2, 3. In this embodiment, in addition the system has a risk checker module 130 to receive a notification from the mapper in case the corresponding predefined risk level indicates a need for therapeutic intervention, and to check 1710 for each risk factor of the current set 210, 220, etc. (cf. loop 1700) if the respective risk factor exceeds a corresponding maximum value, or falls below a corresponding minimum value.

If the respective risk factor exceeds the corresponding maximum value, or falls below the corresponding minimum value, the risk checker retrieves 1720 one or more suggestions for interventions from an intervention database 400. The intervention database 400 can be an integral component of the computer system 100, or it can be a component on a remote computing device which is accessible by the risk checker. The retrieved suggestions for intervention are appropriate to reduce the predicted risk for the patient when being applied to said patient. The risk checker 130 finally provides 1800 the retrieved one or more suggestions 410 to the patient 10 and/or to said medically trained person.

Table 2 illustrates an example of an intervention database with intervention suggestions for the behavioral risk factors of the minimum viable set in situations when the respective risk factor value is outside the healthy range (too high or too low).

*Table 2 - behavioral risk factors and associated interventions*

| # | Risk Factor Name | Interventions when risk factor value is too high | Interventions when risk factor value is too low |
|---|---|---|---|
| 1 | Waist circumference | • Do at least 150 min/week of moderate-intensity, or 75 min/week of vigorous-intensity physical activity<br>• Try to do 3x20 min/week of HITT workout<br>• Assess diet with nutritional expert<br>• Start measuring nutrient intake and reduce calories, reduce saturated fats<br>• Reduce alcohol and fast food and processed food intake | • Seek medical care at GP / lifestyle physician / psychologist |
| 2 | Systolic blood pressure, Hypertension | • Assess and reduce stress<br>• Reduce salt intake<br>• Reduce caffeine intake<br>• Do at least 150 min/week of moderate-intensity, or 75 min/week of vigorous-intensity physical activity • Try to do 3x20 min/week of HITT workout<br>• Assess diet with nutritional expert<br>• Limit alcohol intake<br>• Quit smoking<br>• Lose weight | • Seek medical care at GP/ cardiologist / lifestyle physician |
| 3 | No. of cigarettes daily, smoking | • Quit smoking<br>• Seek smoking cessation care group/facility<br>• Try nicotine patches instead | / |
| 4 | Alcohol with meals Yes, Alcohol with meals: It varies | • Stop having alcohol with meals<br>• Reduce daily/weekly glasses of alcohol<br>• Try to always pause 1-2 days between alcohol consumption | / |

(continued)

| # | Risk Factor Name | Interventions when risk factor value is too high | Interventions when risk factor value is too low |
|---|---|---|---|
| 5 | Social visits: About once/week, Social visits: 2-4 times/week, Social visits: About once/month, Social visits: Almost daily, Social visits: Once every few months | / | • Seek out more (virtual) visits with friends whenever they pop-up in your head<br>• Join a music/ sports class<br>• Pick a new hobby that gets you out of home regularly<br>• Join an online community to find friends with similar hobbies<br>• Set fixed weekly visits with friends |
| 6 | Walking pace: Brisk pace, Walking pace: Steady average pace | / | • Try to go for a walk daily<br>• Walk faster<br>• Seek out GP/ orthopedist for walking related issues |

(continued)

| # | Risk Factor Name | Interventions when risk factor value is too high | Interventions when risk factor value is too low |
|---|---|---|---|
| 7 | Sleep problems: Not at all, Sleep problems: Several days | • Try to set an alarm to limit sleep<br>• Seek a GP/psychologist/ lifestyle physician<br><br>• Reduce stress<br>• Seek out friends | • Hang up thick curtains filtering all light in bedroom<br>• Remove TV from bedroom<br>• Turn on a red light in the evenings<br>• Get blue light filtering glasses to wear in the evenings<br>• Set and stick to a bedtime routine every day of week<br>• Don't work past 9pm, don't take your phone/ laptop/tablet to bed<br>• Charge your phone in another room and get an analog alarm<br>• Seek a lifestyle physician<br>• Take multiple breaks<br>• Exercise more<br>• Don't eat too late (aim for 12hrs between dinner & breakfast) |

(continued)

| # | Risk Factor Name | Interventions when risk factor value is too high | Interventions when risk factor value is too low |
|---|---|---|---|
| 8 | Overall health rating: Excellent, Overall health rating: Poor | / | • Seek lifestyle physician<br>• Exercise more<br>• Set and stick to a bedtime routine every day of week<br>• Don't work past 9pm, don't take your phone/laptop/tablet to bed<br>• Seek out more (virtual) visits with friends whenever they pop-up in your head<br>• Join a music/sports class<br>• Pick a new hobby that gets you out of home regularly<br>• Join an online community to find friends with similar hobbies<br>• Set fixed weekly visits with friends<br>• Assess diet with nutritional expert<br>• Avoiding harmful use of alcohol |

(continued)

| # | Risk Factor Name | Interventions when risk factor value is too high | Interventions when risk factor value is too low |
|---|---|---|---|
| 9 | Total cholesterol, LDL cholesterol, Total cholesterol/HDL ratio | • Increase omega-3 fatty acids<br><br>• Reduce saturated fats<br>• Take brisk walks<br>• Consume more raw fruits & vegetables, avoid processed foods<br>• Do at least 150 min/week of moderate-intensity, or 75 min/week of vigorous-intensity physical activity<br>• Try to do 3x20 min/week of HITT workout<br>• Assess diet with nutritional expert<br>• Avoiding harmful use of alcohol<br>• Evaluate Cholesterol lowering drugs with GP<br>• Stop smoking<br>• Lose weight | • Seek lifestyle physician |
| 10 | Other Heart Arrhythmias, Familial CVD | • Seek a cardiologist<br>• Seek lifestyle physician<br>• Get a consultation for drugs e.g., statins<br>• Evaluate surgery with cardiologist | / |

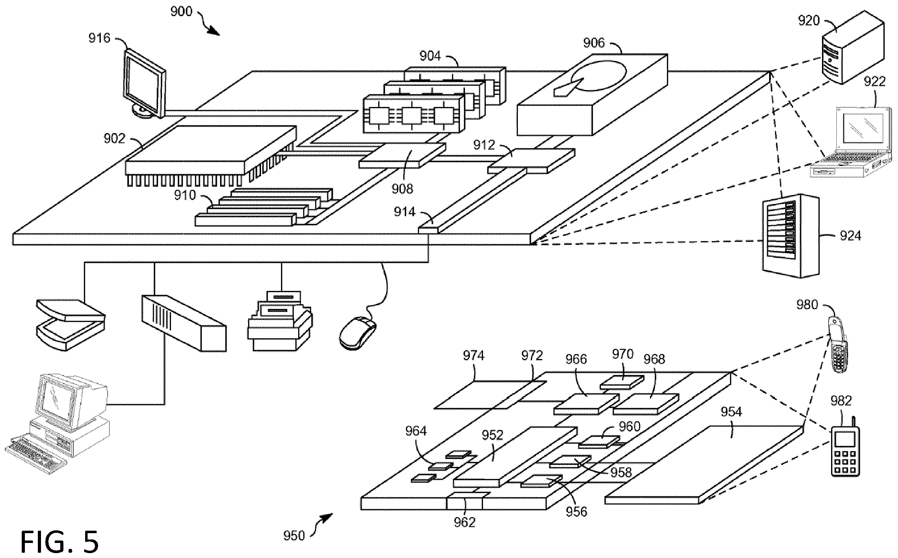

FIG. 5 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, servers, blade servers, mainframes, and other appropriate computers. Generic computer device 900 may correspond to the computer system 100 of FIG. 1. Computing device 950 is intended to represent various forms of mobile devices, such as personal digital assistants, cellular telephones, smart phones, and other similar computing devices. For example, computing device 950 may be used as a GUI frontend for a user to provide test input data and provide them to the computer device 900, and in turn, receive from the computer device, the predicted CVD risk value or notifications with regard to the need of therapeutic interventions. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low speed interface 912 connecting to low speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high speed interface 908. In other implementations, multiple processing units and/or multiple buses may be used, as appropriate, along with multiple memories and types of memory. Also, multiple computing devices 900 may be connected, with each device providing portions of the necessary operations (e.g., as a server bank, a group of blade servers, or a processing device).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high speed controller 908 manages bandwidth-intensive operations for the computing device 900, while the low speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processing units. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provided in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

## Claims

1. A computer-implemented method (1000) for predicting the absolute risk of a patient (10) to develop an atherosclerotic cardiovascular disease within a predefined time interval (p1, p2), the predefined time interval specifying a minimum time period which it takes for the development of said disease, the method comprising:

   receiving (1100) a current set of risk factors associated with the risk of atherosclerotic plaque build-up in the patient's arteries, wherein the current set comprises a set of human-curated risk factors with at least demographic risk (210) factors comprising at least the patient's age and gender, biomarker risk factors (220) comprising at least the patient's waist circumference, systolic blood pressure, total Cholesterol, LDL Cholesterol, and total Cholesterol HDL ratio, comorbidity risk factors (230) comprising at least the patient's hypertension, Sleep problems: Not at all, Sleep problems: Several days, and other Heart Arrhythmias different from Atrial Fibrillations, genetic risk factors (240) comprising at least the patient's familial CVD history, and lifestyle risk factors (250) comprising at least the patient's smoking status, number of cigarettes smoked per day, indicator if alcohol is usually consumed with meals, indicator if alcohol is sometimes consumed with meals, social visits: 2-4 times a week, social visits: about once a week, social visits: about once/month, social visits: almost daily, social visits: once every few months, walking pace: brisk pace, and walking pace: steady average pace, indicator if a self-rated overall health rating is excellent, indicator if a self-rated overall health rating is poor;

providing (1200) the current set as test input to a predictor (110), wherein the predictor has been trained with a training data set (300) including a number of training patient records, the number of training patient records being orders of magnitude greater than the number of risk factors, the training data set including risk factor training values (310 to 350) corresponding to the same set of variables as included in the test input of said patient (10), and serving as training inputs, the training data set further including atherosclerotic cardiovascular disease outcomes (390) of the training patients (90) serving as ground truth, wherein the risk factor training values and the outcomes were obtained over a training data collection period (tc) of at least the length of the predefined time interval, and wherein none of the training patients (90) has or had one of the atherosclerotic cardiovascular diseases at the start of the training data collection period; and

in response to the test input, obtaining (1300) from the predictor (110) a predicted risk value (111) for developing an atherosclerotic cardiovascular disease for said patient (10) within the predefined time interval.

2. The method of claim 1, wherein:

the demographic risk factors (210) further comprise the patient's ethnicity;
the biomarkers risk factors (220) further comprise any of the patient's further weight-related measurements, blood-sample related measurements, heart rate, and glucose;
the comorbidity risk factors (230) further comprise any of the patient's atrial fibrillation, diabetes type 2, diabetes type 1, chronic kidney disease, migraines, rheumatoid arthritis, systemic lupus erythematosus, schizophrenia, bipolar, depression, psychosis, diagnosis or treatment of erectile dysfunction, and atypical antipsychotic medication, inflammation markers, steroids, or medication for any of said comorbidities;
the genetic risk factors (240) further comprise any of the patient's family history of stroke, diabetes, high cholesterol, and high blood pressure;
the lifestyle risk factors (250) further comprise any of the patient's stress, overall health rating, physical activity, and sleep status; and

wherein the risk factors further comprise environmental risk factors (260) with any of the patient's exposure to tobacco smoke, work, housing, and other socio-economic factors.

3. The method of any of the previous claims, further comprising:

mapping (1400) the obtained predicted risk (111) to a corresponding predefined risk level (3);
in case the corresponding predefined risk level (3) indicates a need for therapeutic intervention, notifying the patient (10) and/or a medically trained person accordingly.

4. The method of any of the previous claims, further comprising:

mapping (1400) the obtained predicted risk (111) to a corresponding predefined risk level (3);
in case the corresponding predefined risk level indicates a need for therapeutic intervention:
for each risk factor of the current set:

checking (1710) if the respective risk factor exceeds a corresponding maximum value, or falls below a corresponding minimum value; and
if the respective risk factor exceeds the corresponding maximum value, or falls below the corresponding minimum value, retrieving (1720) from an intervention database (400) one or more suggestions for interventions which, when applied to the patient, are appropriate to reduce the predicted risk for said patient;

providing (1800) the retrieved one or more suggestions (410) to the patient (10) and/or to a medically trained person.

5. The method of any of the previous claims, further comprising:
repeating (1900) the previous steps once per predefined monitoring time interval for said patient.

6. The method of any of the previous claims, wherein a atherosclerotic cardiovascular disease outcome is any of the

following: Coronary/Ischaemic heart disease, Heart attack, Angina, Stroke, Cardiac Arrest, Congestive Heart Failure, Left ventricular failure, Myocardial Infarction, Aortic valve stenosis, Cerebral artery occlusions, Nontraumatic haemorrhages.

7.  The method of any of the previous claims, wherein the predictor is implemented as a LogisticRegression Predictor or as an Extreme Gradient Boosting Predictor.

8.  A computer program product for predicting the absolute risk of a patient (10) to develop a atherosclerotic cardiovascular disease within a predefined time interval, comprising computer-readable instructions that, when loaded into a memory of a computing device and executed by one or more processors of the computing device, cause the computing device to execute the method steps according to any of the previous claims.

9.  A computer system (100) for predicting the absolute risk of a patient (10) to develop an atherosclerotic cardiovascular disease within a predefined time interval (p1, p2), the predefined time interval specifying a minimum time period which it takes for the development of said disease, the system comprising:

    an interface configured to receive a current set of risk factors associated with the risk of atherosclerotic plaque build-up in the patient's arteries, wherein the current set comprises a set of human-curated risk factors with at least demographic risk (210) factors comprising at least the patient's age and gender, biomarker risk factors (220) comprising at least the patient's waist circumference, systolic blood pressure, total Cholesterol, LDL Cholesterol, and total Cholesterol HDL ratio, comorbidity risk factors comprising at least the patient's hypertension, Sleep problems: Not at all, Sleep problems: Several days, and other Heart Arrhythmias different from Atrial Fibrillations, genetic risk factors (240) comprising at least the patient's familial CVD history, and lifestyle risk factors (250) comprising at least the patient's smoking status, number of cigarettes smoked per day, indicator if alcohol is usually consumed with meals, indicator if alcohol is sometimes consumed with meals, social visits: 2-4 times a week, social visits: about once a week, social visits: about once/month, social visits: almost daily, social visits: once every few months, walking pace: brisk pace, and walking pace: steady average pace, indicator if a self-rated overall health rating is excellent, indicator if a self-rated overall health rating is poor; and
    a predictor (110) configured to receive the current set as test input, wherein the predictor has been trained with a training data set (300) including a number of training patient records, the number of training patient records being orders of magnitude greater than the number of risk factors, the training data set including risk factor training values (310 to 350) corresponding to the same set of variables as included in the test input of said patient (10), and serving as training inputs, the training data set further including atherosclerotic cardiovascular disease outcomes (390) of the training patients (90) serving as ground truth, wherein the risk factor training values and the outcomes were obtained over a training data collection period (tc) of at least the length of the predefined time interval, and wherein none of the training patients (90) has or had one of the atherosclerotic cardiovascular diseases at the start of the training data collection period; the predictor (110) further configured to provide, in response to the test input, a predicted risk (111) for developing an atherosclerotic cardiovascular disease for said patient (10) within the predefined time interval.

10. The system of claim 9, wherein the predictor is implemented as a LogisticRegression Predictor or as an Extreme Gradient Boosting Predictor.

11. The system of any of the claims 9 to 10, further comprising:
    a mapper module (120) configured to map the obtained predicted risk (111) to a corresponding predefined risk level (3); and in case the corresponding predefined risk level (3) indicates a need for therapeutic intervention, to notify the patient (10) or a medically trained person accordingly.

12. The system of any of the claims 9 to 10, further comprising:

    a mapper module (120) configured to map the obtained predicted risk (111) to a corresponding predefined risk level (3);
    a risk checker module (130) configured to receive a notification from the mapper module in case the corresponding predefined risk level indicates a need for therapeutic intervention; and to:

        check for each risk factor of the current set if the respective risk factor exceeds a corresponding maximum value, or falls below a corresponding minimum value; and
        if the respective risk factor exceeds the corresponding maximum value, or falls below the corresponding minimum value, to retrieve from an intervention database (400) one or more suggestions for interventions which, when applied to the patient, are appropriate to reduce the predicted risk for said patient; and

further configured to provide the retrieved one or more suggestions (410) to the patient (10) and/or to a medically trained person.

13. The system of any of the claims 9 to 12, further comprising.

a control module (140) configured to trigger the risk prediction by the predictor once per predefined monitoring time interval for said patient.

**Patentansprüche**

1. Ein Computer-implementiertes Verfahren (1000) zur Vorhersage des absoluten Risikos eines Patienten (10), eine atherosklerotische kardiovaskuläre Erkrankung innerhalb eines vordefinierten Zeitintervalls (p1, p2) zu entwickeln, wobei das vordefinierte Zeitintervall eine minimale Zeitspanne angibt, die für die Entwicklung der Erkrankung benötigt wird, wobei das Verfahren umfasst:

Empfangen (1100) eines aktuellen Satzes von Risikofaktoren, die mit dem Risiko einer atherosklerotischen Plaquebildung in den Arterien des Patienten assoziiert sind, wobei der aktuelle Satz einen Satz von menschlich kuratierten Risikofaktoren umfasst, wobei mindestens demographische Risikofaktoren (210) mindestens das Alter und das Geschlecht des Patienten umfassen, Biomarker-Risikofaktoren (220), die mindestens den Taillenumfang des Patienten, den systolischen Blutdruck, das Gesamtcholesterin, das LDL-Cholesterin und das Gesamtcholesterin-HDL-Verhältnis umfassen, Begleiterkrankungs-Risikofaktoren (230), die mindestens den Bluthochdruck des Patienten umfassen, Schlafprobleme: überhaupt nicht, Schlafprobleme: Mehrere Tage, und andere Herzrhythmusstörungen als Vorhofflimmern, genetische Risikofaktoren (240), die zumindest die familiäre CVD Vorgeschichte des Patienten umfassen, und Lebensstil-Risikofaktoren (250), die zumindest den Raucherstatus des Patienten, die Anzahl der pro Tag gerauchten Zigaretten, den Indikator, ob Alkohol gewöhnlich zu den Mahlzeiten konsumiert wird, den Indikator, ob Alkohol manchmal zu den Mahlzeiten konsumiert wird, soziale Besuche umfassen: 2-4 Mal pro Woche, soziale Besuche: etwa einmal pro Woche, soziale Besuche: etwa einmal pro Monat, soziale Besuche: fast täglich, soziale Besuche: einmal alle paar Monate, Gehtempo: zügiges Tempo und Gehtempo: gleichmäßiges, mittleres Tempo, Indikator, wenn die selbst eingeschätzte Gesamtbewertung des Gesundheitszustands ausgezeichnet ist, Indikator, wenn die selbst eingeschätzte Gesamtbewertung des Gesundheitszustands schlecht ist;
Bereitstellen (1200) des aktuellen Satzes als Testeingabe für einen Prädiktor (110), wobei der Prädiktor mit einem Trainingsdatensatz (300) trainiert wurde, der eine Anzahl von Trainingspatientendatensätzen enthält, wobei die Anzahl der Trainingspatientendatensätze um Größenordnungen größer ist als die Anzahl der Risikofaktoren, wobei der Trainingsdatensatz Risikofaktor-Trainingswerte (310 bis 350) enthält, die demselben Satz von Variablen entsprechen, wie sie in der Testeingabe des Patienten (10) enthalten sind, und als Trainingseingaben dienen, wobei der Trainingsdatensatz ferner atherosklerotische kardiovaskuläre Krankheitsergebnisse enthält (390) der Trainingspatienten (90), die als Grundwahrheit dienen, wobei die Risikofaktor-Trainingswerte und die Ergebnisse über eine Trainingsdatenerfassungsperiode (tc) gewonnen wurden von mindestens der Länge des vordefinierten Zeitintervalls, und wobei keiner der Trainingspatienten (90) eine der atherosklerotischen kardiovaskulären Erkrankungen zu Beginn der Trainingsdatenerfassungsperiode hat oder hatte; und
als Reaktion auf die Testeingabe, Erhalten (1300) eines vorhergesagten Risikowerts (111) für die Entwicklung einer atherosklerotischen kardiovaskulären Erkrankung für den Patienten (10) innerhalb des vordefinierten Zeitintervalls von dem Prädiktor (110).

2. Das Verfahren aus Anspruch 1, wobei:

die demografischen Risikofaktoren (210) auch die ethnische Zugehörigkeit des Patienten umfassen;
die Biomarker-Risikofaktoren (220) ferner weitere gewichtsbezogene Messungen des Patienten, blutprobenbezogene Messungen, Herzfrequenz und Glukose umfassen;
die Begleiterkrankungs-Risikofaktoren (230) ferner Vorhofflimmern, Diabetes Typ 2, Diabetes Typ 1, chronische Nierenerkrankung, Migräne, rheumatoide Arthritis, systemischer Lupus erythematodes, Schizophrenie, bipolare Störungen, Depressionen, Psychosen, Diagnose oder Behandlung von erektiler Dysfunktion und atypische antipsychotische Medikamente, Entzündungsmarker, Steroide oder Medikamente für eine der genannten Komorbiditäten des Patienten umfassen;
die genetischen Risikofaktoren (240) außerdem die familiäre Vorgeschichte des Patienten mit Schlaganfall, Diabetes, hohem Cholesterinspiegel und hohem Blutdruck umfassen;
die Lebensstil-Risikofaktoren (250) außerdem den Stress, die allgemeine Gesundheitsbewertung, die körperliche Aktivität und den Schlafstatus des Patienten umfassen; und

wobei die Risikofaktoren ferner umweltbedingte Risikofaktoren (260) umfassen, mit der Exposition des Patienten gegenüber Tabakrauch, der Arbeit, dem Wohnen und andere sozioökonomischen Faktoren.

3. Das Verfahren nach jeglichem der vorhergehenden Ansprüche umfasst ferner:
Zuordnung (1400) des erhaltenen vorhergesagten Risikos (111) zu einem entsprechenden vordefinierten Risikoniveau (3); falls das entsprechende vordefinierte Risikoniveau (3) auf die Notwendigkeit einer therapeutischen Intervention hinweist, und den Patienten (10) und/oder eine medizinisch ausgebildete Person entsprechend zu informieren.

4. Das Verfahren nach jeglichem der vorhergehenden Ansprüche umfasst ferner:

Zuordnung (1400) des erhaltenen vorhergesagten Risikos (111) zu einem entsprechenden vordefinierten Risikoniveau (3);
wenn das entsprechende vordefinierte Risikoniveau auf die Notwendigkeit eines therapeutischen Eingriffs hinweist:
für jeden Risikofaktor des aktuellen Satzes:

Prüfung (1710), ob der jeweilige Risikofaktor einen entsprechenden Maximalwert überschreitet oder einen entsprechenden Minimalwert unterschreitet; und
wenn der jeweilige Risikofaktor den entsprechenden Maximalwert überschreitet oder den entsprechenden Minimalwert unterschreitet, Abrufen (1720) eines oder mehrerer Vorschläge für Interventionen aus einer Interventionsdatenbank (400), die, wenn sie auf den Patienten angewendet werden, geeignet sind, das vorhergesagte Risiko für den Patienten zu verringern;

Bereitstellen (1800) der abgerufenen einen oder mehreren Vorschläge (410) an den Patienten (10) und/oder an eine medizinisch ausgebildete Person.

5. Das Verfahren nach jeglichem der vorhergehenden Ansprüche umfasst ferner:
Wiederholung (1900) der vorherigen Schritte einmal pro vordefiniertem Überwachungszeitintervall für den Patienten.

6. Das Verfahren nach jeglichem der vorhergehenden Ansprüche umfasst ferner, wobei das Ergebnis einer atherosklerotischen Herz-Kreislauf-Erkrankung eines der folgenden ist: Koronare/ischämische Herzkrankheit, Herzinfarkt, Angina pectoris, Schlaganfall, Herzstillstand, Herzinsuffizienz, Linksherzinsuffizienz, Myokardinfarkt, Aortenklappenstenose, Verschlüsse von Hirnarterien, nicht traumatische Blutungen.

7. Das Verfahren nach jeglichem der vorhergehenden Ansprüche umfasst ferner, wobei der Prädiktor als LogisticRegression-Prädiktor oder als Extreme Gradient Boosting-Prädiktor implementiert ist.

8. Ein Computerprogrammprodukt zur Vorhersage des absoluten Risikos eines Patienten (10), innerhalb eines vordefinierten Zeitintervalls eine atherosklerotische kardiovaskuläre Erkrankung zu entwickeln, umfassend computerlesbare Anweisungen, die, wenn sie in einen Speicher einer Rechenvorrichtung geladen und von einem oder mehreren Prozessoren der Rechenvorrichtung ausgeführt werden, die Rechenvorrichtung veranlassen, die Verfahrensschritte gemäß einem der vorhergehenden Ansprüche auszuführen.

9. Ein Computersystem (100) zur Vorhersage des absoluten Risikos eines Patienten (10), eine atherosklerotische kardiovaskuläre Erkrankung innerhalb eines vordefinierten Zeitintervalls (p1, p2) zu entwickeln, wobei das vordefinierte Zeitintervall eine minimale Zeitspanne angibt, die für die Entwicklung der Erkrankung benötigt wird, wobei das System besteht aus:

einer Schnittstelle, die so konfiguriert ist, dass sie einen aktuellen Satz von Risikofaktoren empfängt, die mit dem Risiko einer atherosklerotischen Plaquebildung in den Arterien des Patienten assoziiert sind, wobei die aktuelle Menge eine Menge von menschlich kuratierten Risikofaktoren mit mindestens demographischen Risikofaktoren (210) umfasst, die mindestens das Alter und das Geschlecht des Patienten umfassen, Biomarker-Risikofaktoren (220), die mindestens den Taillenumfang des Patienten, den systolischen Blutdruck, das Gesamtcholesterin, das LDL-Cholesterin und das Gesamtcholesterin-HDL-Verhältnis umfassen, Begleiterkrankungs-Risikofaktoren, die mindestens den Bluthochdruck des Patienten umfassen, Schlafprobleme: Überhaupt nicht, Schlafprobleme: Mehrere Tage, und andere Herzrhythmusstörungen als Vorhofflimmern, genetische Risikofaktoren (240), die zumindest die familiäre CVD Vorgeschichte des Patienten umfassen, und Lebensstil-Risikofaktoren (250), die zumindest den Raucherstatus des Patienten, die Anzahl der pro Tag gerauchten Zigaretten, den Indikator, ob Alkohol gewöhnlich zu den Mahlzeiten konsumiert wird, den Indikator, ob Alkohol manchmal zu den Mahlzeiten konsumiert wird, soziale Besuche umfassen: 2-4 Mal pro Woche, soziale Besuche: etwa einmal pro Woche, soziale Besuche: etwa einmal pro Monat, soziale Besuche: fast täglich, soziale Besuche: einmal alle paar Monate, Gehtempo: zügiges Tempo und Gehtempo: gleichmäßiges, mittleres Tempo, Indikator, wenn die selbst eingeschätzte Gesamtbewertung des Gesundheitszustands ausgezeichnet ist, Indikator, wenn die selbst eingeschätzte Gesamtbewertung des Gesundheitszustands schlecht ist;

und einen Prädiktor (110), der so konfiguriert ist, dass er die aktuellen Menge als Testeingabe empfängt, wobei der Prädiktor mit einem Trainingsdatensatz (300) trainiert wurde, der eine Anzahl von Trainingspatientendatensätzen enthält, wobei die Anzahl von Trainingspatientendatensätzen um Größenordnungen größer ist als die Anzahl von Risikofaktoren, wobei der Trainingsdatensatz Risikofaktor-Trainingswerte (310 bis 350) enthält, die demselben Satz von Variablen entsprechen wie die in der Testeingabe des Patienten (10) enthalten sind und als Trainingseingaben dienen, wobei der Trainingsdatensatz außerdem Ergebnisse von atherosklerotischen kardiovaskulären Erkrankungen enthält (390) der Trainingspatienten (90), die als Grundwahrheit dienen, wobei die Risikofaktor-Trainingswerte und die Ergebnisse über eine Trainingsdatensammlungsperiode (tc) von mindestens der Länge des vordefinierten Zeitintervalls erhalten wurden, und wobei keiner der Trainingspatienten (90) zu Beginn der Trainingsdatensammlungsperiode eine der atherosklerotischen kardiovaskulären Erkrankungen hat oder hatte; wobei der Prädiktor (110) ferner so konfiguriert ist, dass er in Reaktion auf die Testeingabe ein vorhergesagtes Risiko (111) für die Entwicklung einer atherosklerotischen kardiovaskulären Erkrankung für den Patienten (10) innerhalb des vordefinierten Zeitintervalls bereitstellt.

10. Das System aus Anspruch 9, wobei der Prädiktor als LogisticRegression-Prädiktor oder als Extreme Gradient Boosting-Prädiktor implementiert ist.

11. Das System nach einem Beliebigen der Ansprüche 9 bis 10, das außerdem Folgendes umfasst:

ein Mappermodul (120), das so konfiguriert ist, dass es das erhaltene vorhergesagte Risiko (111) auf ein entsprechendes vordefiniertes Risikoniveau (3) abbildet; und,
falls das entsprechende vordefinierte Risikoniveau (3) die Notwendigkeit eines therapeutischen Eingriffs anzeigt, den Patienten (10) oder eine medizinisch ausgebildete Person entsprechend benachrichtigt.

12. Das System nach einem Beliebigen der Ansprüche 9 bis 10, das außerdem Folgendes umfasst:

ein Mappermodul (120), das so konfiguriert ist, dass es das erhaltene vorhergesagte Risiko (111) einem entsprechenden vordefinierten Risikoniveau (3) zuordnet;
ein Risikokontrollmodul (130), das so konfiguriert ist, dass es eine Benachrichtigung von dem Mappermodul empfängt, wenn das entsprechende vordefinierte Risikoniveau die Notwendigkeit eines therapeutischen Eingriffs anzeigt; und um:

für jeden Risikofaktor der aktuellen Menge zu prüfen, ob der jeweilige Risikofaktor einen entsprechenden Höchstwert überschreitet oder einen entsprechenden Mindestwert unterschreitet;
und wenn der jeweilige Risikofaktor den entsprechenden Maximalwert überschreitet oder den entsprechenden Minimalwert unterschreitet, aus einer Interventionsdatenbank (400) einen oder mehrere Vorschläge für Interventionen, die, wenn sie auf den Patienten angewendet werden, geeignet sind, das vorhergesagte Risiko für den Patienten zu verringern; und

des Weiteren so konfiguriert, dass die abgerufenen ein oder mehreren Vorschläge (410) dem Patienten (10) und/oder einer medizinisch ausgebildeten Person zur Verfügung gestellt werden.

13. Das System nach einem Beliebigen der Ansprüche 9 bis 12, das außerdem Folgendes umfasst:
ein Steuermodul (140), das so konfiguriert ist, dass es die Risikovorhersage durch den Prädiktor einmal pro vordefiniertem Überwachungszeitintervall für den Patienten auslöst.

**Revendications**

1. Procédé implémenté par ordinateur (1000) pour prédire le risque absolu d'un patient (10) de développer une maladie cardiovasculaire athérosclérotique au cours d'un intervalle de temps prédéfini (p1, p2), l'intervalle de temps prédéfini spécifiant un laps de temps minimal que prend le développement de ladite maladie, le procédé comprenant :fr

   la réception (1100) d'un ensemble actuel de facteurs de risque associés au risque d'accumulation de plaque athéroscléreuse dans les artères du patient, l'ensemble actuel comprenant un ensemble de facteurs de risque d'origine humaine avec au moins des facteurs de risque démographiques (210) comprenant au moins l'âge et le sexe du patient, des facteurs de risque à biomarqueur (220) comprenant au moins le tour de taille du patient, la pression sanguine systolique, le cholestérol total, le cholestérol LDL, et le rapport cholestérol total-HDL, des facteurs de risque de comorbidité (230) comprenant au moins une hypertension du patient, des problèmes de sommeil : pas du tout, des problèmes de sommeil : plusieurs jours, et d'autres arythmies cardiaques différentes de fibrillations auriculaires, des facteurs de risque génétiques (240) comprenant au moins des antécédents familiaux de MCV du patient, et des facteurs de risque de mode de vie (250) comprenant au moins le statut de tabagisme du patient, le nombre de cigarettes fumées par jour, un indicateur indiquant si de l'alcool est habituellement consommé avec les repas, un indicateur indiquant si de l'alcool est parfois consommé avec les repas, des visites sociales : 2 à 4 fois par semaine, des visites sociales : environ une fois par semaine, des visites sociales : environ une fois par mois, des visites sociales : pratiquement chaque jour, des visites sociales : une fois tous les quelques mois, l'allure de marche : allure rapide, et l'allure de marche : allure moyenne régulière, un indicateur indiquant si une auto-évaluation de l'état de santé général est excellente, un indicateur indiquant si une auto-évaluation de l'état de santé général est mauvaise ;
   la fourniture (1200) de l'ensemble actuel en guise d'entrée de test à un prédicteur (110), le prédicteur ayant été entraîné avec un ensemble de données d'entraînement (300) comportant un certain nombre d'enregistrements de patient d'entraînement, le nombre d'enregistrements de patient d'entraînement étant d'ordres de grandeur supérieurs au nombre de facteurs de risque, l'ensemble de données d'entraînement comportant des valeurs d'entraînement de facteur de risque (310 à 350) correspondant au même ensemble de variables que celles incluses dans l'entrée de test dudit patient (10), et servant d'entrées d'entraînement, l'ensemble de données d'entraînement comportant en outre des issues de maladie cardiovasculaire athérosclérotique (390) des patients d'entraînement (90) servant de réalité de terrain, les valeurs d'entraînement de facteur de risque et les issues ayant été obtenues sur une période de collecte de données d'entraînement (tc) d'au moins la longueur de l'intervalle de temps prédéfini, et aucun des patients d'entraînement (90) n'ayant ou n'ayant eu l'une des maladies cardiovasculaires athérosclérotiques au début de la période de collecte de données d'entraînement ; et
   en réponse à l'entrée de test, l'obtention (1300) par le prédicteur (110) d'une valeur de risque prédit (111) de développement d'une maladie cardiovasculaire athérosclérotique pour ledit patient (10) au cours de l'intervalle de temps prédéfini.

2. Procédé selon la revendication 1, dans lequel :

   les facteurs de risque démographiques (210) comprennent en outre l'appartenance ethnique du patient ;
   les facteurs de risque à biomarqueur (220) comprennent en outre l'une quelconque parmi d'autres mesures liées au poids, des mesures liées à des échantillons de sang, la fréquence cardiaque, et la glycémie, du patient ;
   les facteurs de risque de comorbidité (230) comprennent en outre l'un quelconque parmi une fibrillation auriculaire, un diabète de type 2, un diabète de type 1, une maladie rénale chronique, des migraines, une polyarthrite rhumatoïde, un lupus érythémateux disséminé, une schizophrénie, une bipolarité, une dépression, une psychose, un diagnostic ou traitement de dysfonctionnement érectile, et une médication antipsychotique atypique, des marqueurs d'inflammation, des stéroïdes, ou une médication pour l'une quelconque desdites comorbidités, du patient ;
   les facteurs de risque génétiques (240) comprennent en outre l'un quelconque parmi des antécédents familiaux d'accident vasculaire cérébral, de diabète, de cholestérol élevé, et de pression sanguine élevée, du patient ;
   les facteurs de risque de mode de vie (250) comprennent en outre l'un quelconque parmi le stress, l'évaluation de l'état de santé général, l'activité physique et l'état du sommeil, du patient ; et
   dans lequel les facteurs de risque comprennent en outre des facteurs de risque environnementaux (260) avec l'un quelconque parmi une exposition à de la fumée de tabac, le travail, le logement et d'autres facteurs socio-économiques, du patient.

3. Procédé selon l'une quelconque des revendications précédentes, comprenant en outre :

le mappage (1400) du risque prédit obtenu (111) à un niveau de risque prédéfini correspondant (3) ;
dans un cas où le niveau de risque prédéfini correspondant (3) indique une nécessité d'intervention thérapeutique, la notification du patient (10) et/ou d'une personne médicalement entraînée, en conséquence.

4. Procédé selon l'une quelconque des revendications précédentes, comprenant en outre :

le mappage (1400) du risque prédit obtenu (111) à un niveau de risque prédéfini correspondant (3) ;
dans un cas où le niveau de risque prédéfini correspondant indique une nécessité d'intervention thérapeutique :
pour chaque facteur de risque de l'ensemble actuel :

le fait de vérifier (1710) si le facteur de risque respectif dépasse une valeur maximale correspondante, ou descend en dessous d'une valeur minimale correspondante ; et
si le facteur de risque respectif dépasse la valeur maximale correspondante, ou descend en dessous de la valeur minimale correspondante, la récupération (1720) auprès d'une base de données d'intervention (400) d'une ou plusieurs suggestions pour des interventions qui, lorsqu'elles sont appliquées au patient, sont appropriées pour réduire le risque prédit pour ledit patient ;
la fourniture (1800) de la ou des suggestions récupérées (410) au patient (10) et/ou à une personne médicalement entraînée.

5. Procédé selon l'une quelconque des revendications précédentes, comprenant en outre :
la répétition (1900) des étapes précédentes une fois par intervalle de temps de surveillance prédéfini pour ledit patient.

6. Procédé selon l'une quelconque des revendications précédentes, dans lequel une issue de maladie cardiovasculaire athérosclérotique est l'une quelconque des suivantes : cardiopathie coronarienne/ischémique, crise cardiaque, angine de poitrine, accident vasculaire cérébral, arrêt cardiaque, insuffisance cardiaque congestive, insuffisance ventriculaire gauche, infarctus du myocarde, sténose de la valve aortique, occlusions artérielles cérébrales, hémorragies non traumatiques.

7. Procédé selon l'une quelconque des revendications précédentes, dans lequel le prédicteur est implémenté sous la forme d'un prédicteur LogisticRegression ou d'un prédicteur Extrême Gradient Boosting.

8. Produit programme informatique pour prédire le risque absolu d'un patient (10) de développer une maladie cardiovasculaire athérosclérotique au cours d'un intervalle de temps prédéfini, comprenant des instructions lisibles par ordinateur qui, lorsqu'elles sont chargées dans une mémoire d'un dispositif informatique et exécutées par un ou plusieurs processeurs du dispositif informatique, amènent le dispositif informatique à exécuter les étapes de procédé selon l'une quelconque des revendications précédentes.

9. Système informatique (100) permettant de prédire le risque absolu d'un patient (10) de développer une maladie cardiovasculaire athérosclérotique au cours d'un intervalle de temps prédéfini (p1, p2), l'intervalle de temps prédéfini spécifiant un laps de temps minimal que prend le développement de ladite maladie, le système comprenant :

une interface configurée pour recevoir un ensemble actuel de facteurs de risque associés au risque d'accumulation de plaque athéroscléreuse dans les artères du patient, l'ensemble actuel comprenant un ensemble de facteurs de risque d'origine humaine avec au moins des facteurs de risque démographiques (210) comprenant au moins l'âge et le sexe du patient, des facteurs de risque à biomarqueur (220) comprenant au moins le tour de taille du patient, la pression sanguine systolique, le cholestérol total, le cholestérol LDL, et rapport cholestérol total-HDL, des facteurs de risque de comorbidité comprenant au moins une hypertension du patient, des problèmes de sommeil : pas du tout, des problèmes de sommeil : plusieurs jours, et d'autres arythmies cardiaques différentes de fibrillations auriculaires, des facteurs de risque génétiques (240) comprenant au moins des antécédents familiaux de MCV du patient, et des facteurs de risque de mode de vie (250) comprenant au moins le statut de tabagisme du patient, le nombre de cigarettes fumées par jour, un indicateur indiquant si de l'alcool est habituellement consommé avec les repas, un indicateur indiquant si de l'alcool est parfois consommé avec les repas, des visites sociales : 2 à 4 fois par semaine, des visites sociales : environ une fois par semaine, des visites sociales : environ une fois par mois, des visites sociales : pratiquement chaque jour, des visites sociales : une fois tous les quelques mois, l'allure de marche : allure rapide, et l'allure de marche : allure moyenne régulière, un indicateur indiquant si une auto-évaluation de l'état de santé général est excellente, un indicateur indiquant si une auto-évaluation de l'état de santé général est mauvaise ; et
un prédicteur (110) configuré pour recevoir l'ensemble actuel en guise d'entrée de test, le prédicteur ayant été entraîné avec un ensemble de données d'entraînement (300) comportant un certain nombre d'enregistrements de patient d'entraînement, le nombre d'enregistrements de patient d'entraînement étant d'ordres de grandeur supérieurs au nombre de facteurs de risque, l'ensemble de données d'entraînement comportant des valeurs d'entraînement de facteur de risque (310 à 350) correspondant au même ensemble de variables que celles incluses dans l'entrée de test dudit patient (10), et servant d'entrées d'entraînement, l'ensemble de données d'entraînement comportant en outre des issues de maladie cardiovasculaire athérosclérotique (390) des patients d'entraînement (90) servant de réalité de terrain, les valeurs d'entraînement de facteur de risque et les issues ayant été obtenues sur une période de collecte de données d'entraînement (tc) d'au moins la longueur de l'intervalle de temps prédéfini, et aucun des patients d'entraînement (90) n'ayant ou n'ayant eu l'une des maladies cardiovasculaires athérosclérotiques au début de la période de collecte de données d'entraînement ; le prédicteur (110) configuré en outre pour fournir, en réponse à l'entrée de test, un risque prédit (111) de développement de maladie cardiovasculaire athérosclérotique pour ledit patient (10) au cours de l'intervalle de temps prédéfini.

**10.** Système selon la revendication 9, dans lequel le prédicteur est implémenté sous la forme d'un prédicteur LogisticRegression ou d'un prédicteur Extrême Gradient Boosting.

**11.** Système selon l'une quelconque des revendications 9 à 10, comprenant en outre :
un module mappeur (120) configuré pour mapper le risque prédit obtenu (111) à un niveau de risque prédéfini correspondant (3) ; et dans un cas où le niveau de risque prédéfini correspondant (3) indique une nécessité d'intervention thérapeutique, pour notifier le patient (10) ou une personne médicalement entraînée en conséquence.

**12.** Système selon l'une quelconque des revendications 9 à 10, comprenant en outre :

un module mappeur (120) configuré pour mapper le risque prédit obtenu (111) à un niveau de risque prédéfini correspondant (3) ;
un module vérificateur de risque (130) configuré pour recevoir une notification provenant du module mappeur dans un cas où le niveau de risque prédéfini correspondant indique une nécessité d'intervention thérapeutique ; et pour :

vérifier pour chaque facteur de risque de l'ensemble actuel si le facteur de risque respectif dépasse une valeur maximale correspondante, ou descend en dessous d'une valeur minimale correspondante ; et si le facteur de risque respectif dépasse la valeur maximale correspondante, ou descend en dessous de la valeur minimale correspondante, pour récupérer auprès d'une base de données d'intervention (400) une ou plusieurs suggestions pour des interventions qui, lorsqu'elles sont appliquées au patient, sont appropriées pour réduire le risque prédit pour ledit patient ; et configuré en outre pour fournir la ou des suggestions récupérées (410) au patient (10) et/ou à une personne médicalement entraînée.

**13.** Système selon l'une quelconque des revendications 9 à 12, comprenant en outre :
un module de commande (140) configuré pour déclencher la prédiction de risque par le prédicteur une fois par intervalle de temps de surveillance prédéfini pour ledit patient.

FIG. 1

1000

1900

receiving current risk factors of a patient ⌐ 1100

providing risk factors as test input to predictor ⌐ 1200

obtaining predicted risk ⌐ 1300

mapping predicted risk to risk level ⌐ 1400

NO ◁ need-
for-intervention
indicator? ▷ ⌐ 1500

YES

sending notification ⌐ 1600

for each risk factor: ⌐ 1700

1710
NO ◁ risk factor
outside predefined
range? ▷

1720
YES

retrieving suggestion(s) for interventions ⌐

providing retrieved suggestion(s) to user ⌐ 1800

# FIG. 2

training data
collection period
tc

$p_1$

$p_2$

$tc_s$       $tc_e$   $tp_1$      $tp_2$            time

# FIG. 3

11-1

11-10

11-12   11-11

1. What is your age?      0 ■————————

2. What is you biological sex?      ◉ female    ○ male

3. What is your systolic blood pressure?      0 ■————————

4. What is your waist circumference?      0 ■————————

5. Have you got sleep problems? – Not at all      ◉ yes    ○ no

6. Have you got sleep problems? – Several days      ◉ yes    ○ no

7. Do you smoke?      ◉ yes    ○ no

8. How many cigarettes a day?      0 ■————————

9. ...

# FIG. 4

FIG. 5

## REFERENCES CITED IN THE DESCRIPTION

**Patent documents cited in the description**

- EP 2864793 A1

- RU 2573499 C1

**Non-patent literature cited in the description**

- **ALAA et al.** *Cardiovascular disease risk prediction using automated machine learning: A prospective study of 423,604 UK Biobank participants, https://journals.plos.org/plosone/article?id=10.1371/journal.pone.0213653*
- **COLLINS et al.** A New Initiative on Precision Medicine. *N Engl J Med,* 2015, vol. 372, 793-795

- **PICAR et al.** Integration strategies of multi-omics data for machine learning analysis. *Computational and Structural Biotechnology Journal,* 2021, vol. 19, 3735-3746, https://www.sciencedirect.eom/science/article/pii/S2001037021002683#b0025
- **HASIN et al.** Multi-omics approaches to disease. *Genome Biology,* 2017, vol. 18 (1
- **RALPH B. D'AGOSTINOSR et al.** The Framingham Heart Study. *Circulation,* 22 January 2008, vol. 117, 743-753, https://doi.org/10.1161/CIRCULATIONA-HA.107.699579